(19) Europäisches Patentamt
European Patent Office
Office européen des brevets

(11) EP 0 797 564 B1

(12) EUROPEAN PATENT SPECIFICATION

(45) Date of publication and mention of the grant of the patent: **27.06.2001 Bulletin 2001/26**

(21) Application number: **95936521.4**

(22) Date of filing: **18.10.1995**

(51) Int Cl.[7]: **C07C 69/734**, C07C 67/343, C07C 67/08, C07C 67/303, C07C 33/26, C07C 29/147, C07D 307/12, C07C 43/23, C07D 313/04, A61K 31/34, A61K 31/225

(86) International application number: **PCT/EP95/04096**

(87) International publication number: **WO 97/14670 (24.04.1997 Gazette 1997/18)**

# (54) LIGNANS, A PROCESS FOR THEIR PRODUCTION AND PHARMACEUTICAL COMPOSITIONS AND USES THEREOF

LIGNANE, VERFAHREN ZU IHRER HERSTELLUNG, IHRE PHARMAZEUTISCHE COMPOSITIONEN UND ANWENDUNGEN

LIGNANES, PROCEDE DE PRODUCTION DE CEUX-CI, COMPOSITIONS PHARMACEUTIQUES LES CONTENANT, ET LEURS UTILISATIONS

(84) Designated Contracting States:
**AT BE CH DE DK ES FR GB GR IE IT LI LU MC NL PT SE**

(43) Date of publication of application:
**01.10.1997 Bulletin 1997/40**

(73) Proprietor: **KANOLDT ARZNEIMITTEL GMBH
89420 Höchstädt (DE)**

(72) Inventors:
- **BOOS, Günther
D-89423 Gundelfingen (DE)**
- **Schöttner, Matthias
D-95448 Bayreuth (DE)**
- **Gansser, Dietmar
D-30966 Hemmingen (DE)**
- **Spiteller, Gerhard
D-95445 Bayreuth (DE)**

(74) Representative: **Grünecker, Kinkeldey, Stockmair & Schwanhäusser Anwaltssozietät
Maximilianstrasse 58
80538 München (DE)**

(56) References cited:
- **PATENT ABSTRACTS OF JAPAN vol. 14, no. 197 (C-0712), 23 April 1990 & JP,A,02 040323 (TSUMURA & CO), 9 February 1990,**
- **PATENT ABSTRACTS OF JAPAN vol. 13, no. 553 (C-663), 8 December 1989 & JP,A,01 228928 (TSUMURA & CO), 12 September 1989,**
- **ERIC BROWN et al, "The syntheses of (R)-(+)-beta-vanillyl- butyrolactone and of chiral lignans therefrom", HETEROCYCLES, vol. 26, no. 5, pages 1169 to 1172**

EP 0 797 564 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

The present invention relates to a process for the production of lignans as well as novel lignans which may be produced by this process. It furthermore concerns pharmaceutical compositions containing those lignans which find use as human sex hormone binding inhibitors, anti-tumour agents and agents against prostate cancer.

It is known that lignans can be separated from plant extracts, such as from Coniferae (e.g. Spruce), Magnolia (e.g. Magnolia Fargesii), and Nettle root. It is also known that such lignans may display a wide variety of physiological effects in mammals. Examples of this activity include fertility enhancement when acting as blood platelet activating factors, (O' Neill, PCT patent application no. WO/90 13299), spasmolytic activity when used as anti-hiccough agents, (Matsuura *et al, Phytochem.,* vol. 24, p. 626, 1985), and interference with Human Sex Hormone-Binding Globulin (SHBG) (Ganßer and Spiteller, *Z. Naturforsch.* 50c, p. 98, 1995).

In regard to the development of useful pharmaceuticals comprising lignans, it was naturally necessary to have the active compounds available in considerable quantities, and the problem of realising a synthetic route to lignans thus arose. Morimoto *et al* developed an asymmetric synthesis to several lignans using chiral bisphosphine ligands and Rhodium (I) catalysed asymmetric hydrogenation. In this way (-)-matairesinol and (-)-anhydrosecoisolariciresinol were produced, (*Heterocycles,* vol. 33, p. 435, 1992), however such methods are not industrially practical in terms of cost, time and yield. An oxidative coupling reaction has been used to produce the lignans enterolactone and wikstromol (J. Belletire *et al*, *Tet. Lett.,* vol. 27, p. 127, 1986 and *J. Org. Chem.*, vol. 53, p. 4724, 1988). This method has been applied in several Japanese patent applications for producing lignans of the type isolable from Nettle root (JP-A-88 186853, JP-A-88 250002, JP-A-88 53550). These lignans are indicated to be useful in immuno-suppressant, cardiotonic and anti-tumour compositions respectively. However, these processes necessitate the use of protection groups on the phenol residues, and must be carried out under strict anhydrous conditions in an inert atmosphere, considerably limiting the available yield of pure product. Further methods of production using a Stobbe condensation have been attempted (Matsuura *et al*, *Phytochem.* vol. 24, p. 626, 1985, Brown, *et al*, *Heterocycles,* vol. 26, p. 1169, 1987, and Bambagotti-Alberti, *et al*, *Heterocycles,* vol. 27, p. 2185, 1988) using sodium alcoholate, lithium alcoholate and sodium hydride as bases, respectively. However, condensation with a second aromatic group was achieved through laborious methods involving the use of protection groups on the phenol residues.

Thus, before the achievement of the present invention the problems still remained of determining which particular lignans (especially those isolable from Nettle root), possessed significant interference properties with SHBG, and furthermore of determining the strength of this activity, and the pharmaceutical effects of the compounds.

Additionally an economical and industrially viable process route into these compounds was required before pharmaceuticals comprising these compounds could be effectively produced.

Thus an objective of the present invention is to develop a novel synthesis of lignans, so that they may be produced more easily and on a more commercially viable scale.

A further objective is to provide new lignans which were not previously obtainable from natural sources and could also find use as pharmaceuticals.

Furthermore, the intent was to provide pharmaceutical compositions containing the lignans, especially those which act as SHBG inhibitors. Therefore, further objectives include the determination of the activities of the lignans, and the formation of appropriate pharmaceuticals. A related further objective was to develop the use of the lignans in the treatment of particular ailments.

In attempting to solve these problems, the present inventors developed a new mode of synthesis for lignans, making use of two consecutive Stobbe condensations, using lithium alcoholate as a base. This method is particularly advantageous as it does not require the use of protection groups, nor are such rigorous anhydrous, inert-atmosphere conditions needed. Thus, the process affords a quicker, more simple route into the target compounds, which can be produced in improved yield and purity than has thus far been disclosed in the prior art. Furthermore non-symmetric lignans are also easily produced.

Through the use of this process, new lignans, not previously isolated from natural sources were produced, which were determined to be active as SHBG inhibitors. Furthermore, pharmaceutical compositions were developed comprising these compounds, and it was determined that the lignans have a beneficial pharmaceutical effect when used against tumours and prostate cancer.

Therefore, according to the present invention a process for the production of compounds of formula (I) is provided:

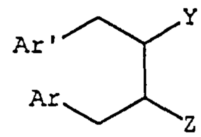

(I)

wherein Ar and Ar' may be the same or different and are represented by groups of the following formula:

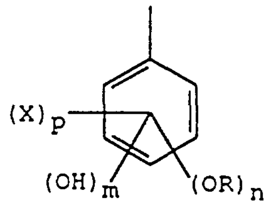

wherein X is a halogen atom, a $C_1$ to $C_6$ alkyl group, an amino group, a nitro group, an acyl group ($CH_3COO$-), a carboxylic acid group or a $C_1$ to $C_6$ alkyl ester of a carboxylic acid, a phenyl group, or a phenyl group substituted with any of the above groups; R is a $C_1$ to $C_6$ alkyl group; and n, m and p are integers of 0 to 5, provided that the sum of n, m and p does not exceed 5; and Y and Z may be the same or different and represent COOH, COOR', $CH_2OH$, or $CH_2OR'$ groups or taken together may represent any groups of the following formulae:

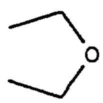 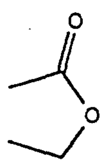 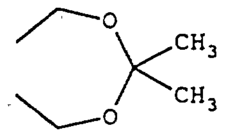

comprising the steps of:

(1) Reacting a compound of formula (Ia):

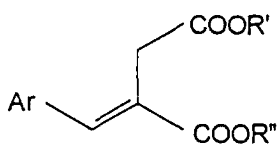

(Ia)

with a lithium/R'OH, or a lithium/R' 'OH mixture in the presence of Ar'-CHO, wherein R' and R" may be the same or different and are each $C_{1-6}$ alkyl or phenyl, to form an intermediate compound (Ib),

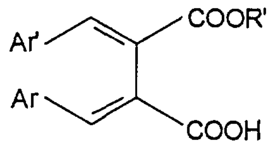

(Ib)

(2) Reducing compounds of formula (Ib), preferably by using hydrogen and a palladium/carbon catalyst, to produce compounds of formula (Ic):

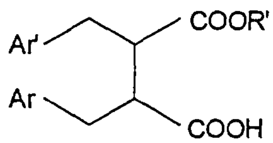

(Ic)

and then optionally performing a further step (3), (6) or (7), or a sequence of steps (3) → (4) or (3) →(4) → (5) given below:

(3) Subjecting compounds (Ic) obtained in step (2) to esterification, preferably by using R' 'OH and sulfuric acid, to produce compounds of formula (II):

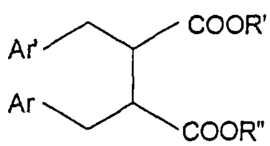

(II)

(4) Reducing the compounds (II) obtained in step (3), preferably by using $LiA1H_4$, to form compounds (III):

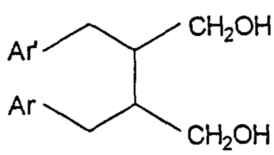

(III)

(5) Subjecting compounds of formula (III) obtained in step (4) to a cyclisation reaction, preferably by using $HClO_4$, to produce compounds of formula (IV):

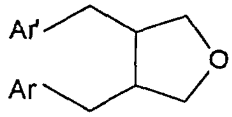

(IV)

(6) Subjecting compounds of formula (Ic) obtained in step (2) to a cyclisation reaction, preferably by using $LiA1H_4$ followed by hydrochloric acid, to form compounds of formula (V):

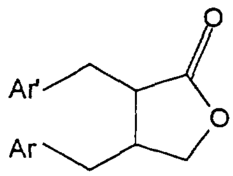

(V)

(7) Subjecting compounds of formula (Ic) obtained in step (2) to an acetal forming reaction in the presence of an acid, to form compounds of formula (III'):

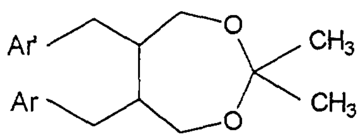

(III')

It should be noted that when groups such as

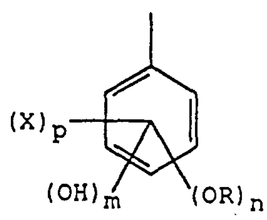

are mentioned it is intended to indicate that they are bound to the general formula by the hanging bond(s) indicated.

Furthermore, lignan compounds of the following formula (I') are provided in the invention:

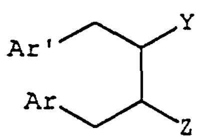

(I')

wherein Ar and Ar' may be the same or different and are represented by groups of the following formula:

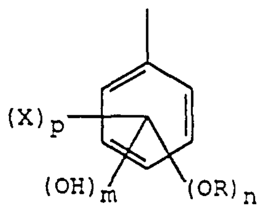

wherein X is -a halogen atom, a $C_1$ to $C_6$ alkyl group, an amino group, a nitro group, an acyl group ($CH_3COO$-), a carboxylic acid group or a $C_1$ to $C_6$ alkyl ester of a carboxylic acid, a phenyl group, or a phenyl group substituted with any of the above groups; R is a $C_1$ to $C_6$ alkyl group; and n, m and p are integers of 0 to 5, provided that the sum of n, m and p does not exceed 5; and Y and Z may be the same or different and represent COOH, COOR', $CH_2OH$, or $CH_2OR'$ groups or taken together may represent any groups of the following formulae:

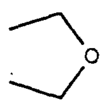

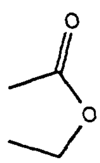

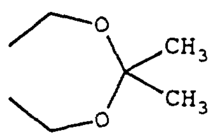

wherein, compounds having p = 0, (m + n) = 3 or less and R = Me are not claimed, except where Ar' is a group of formula:

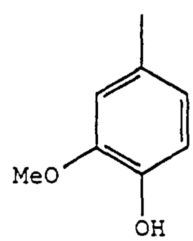

and either:

Ar is a group of the following formula:

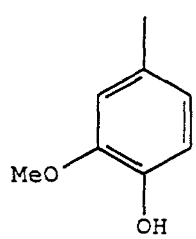

and Y and Z are both COOR' groups, or together form group of formula

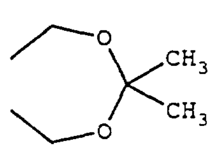

or:

Ar is a group of formula:

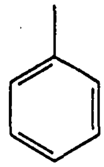

and Y and Z are both COOR' groups, or both a $CH_2OH$ group, or together form a group of formula:

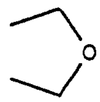

wherein when Y and Z are COOR', the groups R' may be the same or different and are $C_1$ to $C_6$ alkyl groups or phenyl groups.

Additionally, the invention provides the use of compounds of formula (I) in the manufacture of medicaments effective as human sex hormone-binding globuline inhibitors, anti-tumour agents and agents against prostate cancer. Furthermore pharmaceutical compositions comprising compounds (I') are provided.

A preferred process of the present invention, is one in which compounds of the formula (I) are produced in which Y and Z are groups of formula COOR' and COOR", i.e. compounds of formula (II):

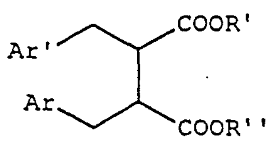

(II)

in which Ar, Ar', R' and R" have the same meanings as described above. A preferred route from compounds (I c) into compounds (II) comprises the following step :

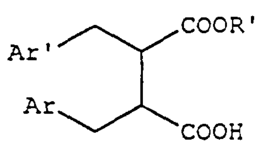

(Ic)

(3) Subjecting compounds (Ic) to esterification, to produce compounds of formula (II).

The conditions and reagents for effecting the reduction and esterification steps are not particularly limited, however, for the step of reduction, hydrogen on a palladium/carbon catalyst is particularly preferred, and for the esterification step the use of R" OH in the presence of sulphuric acid is preferable. The reduction and esterification steps can be carried out in the reverse order if desired.

A further preferred process of the present invention, is one in which compounds of the formula (I) are produced in which both Y and Z are $CH_2OH$ groups, i.e. compounds of formula (III):

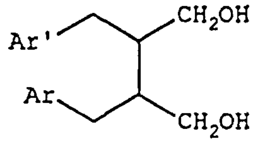

(III)

in which Ar and Ar' have the same meanings as described above. A preferred route from compounds (I c) into compounds (III) comprises step (3) as described above and the additional step:

(4) reducing compounds (II) to form compounds (III).

The conditions and reagents for effecting this reduction step are not particularly limited, however, it is most preferably carried out using $LiAlH_4$. Compounds (III) can additionally be transformed into compounds (III'), if so required, by reaction with acetone in the presence of acid (step (7)):

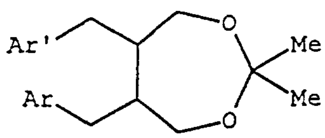

(III')

Another preferred process of the present invention, is one in which compounds of the formula (I) are produced in which Y and Z taken together represent a group of formula:

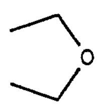

i.e., compounds of formula (IV):

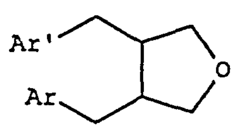

(IV)

in which Ar and Ar' have the same meanings as described above. A preferred route from compounds (Ic) into compounds (IV) comprises steps (3) and (4), as described above and the additional step:

(5) Subjecting compounds of formula (III) to a cyclisation reaction, to produce compounds of formula (IV).

The conditions and reagents for effecting this reduction step are not particularly limited, however, it is most preferably carried out using $HClO_4$. Other acids and also $Al_2O_3$ can be used if desired.

A still further preferred process of the present invention, is one in which compounds of the formula (I) are produced, in which Y and Z taken together represent a group of formula:

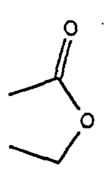

i.e., compounds of formula (V):

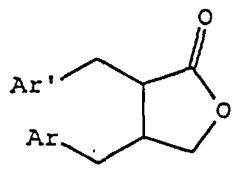

(V)

in which Ar and Ar' have the same meanings as described above. A preferred route from compounds (Ic) into compounds (V) comprises the additional step:

(6) Subjecting compounds of formula (Ic) to a cyclisation reaction to form compounds of formula (V);

The conditions and reagents for effecting this cyclisation step are not particularly limited, however, it is most preferably carried out using $LiAlH_4$, followed by the application of hydrochloric acid.

When p > 0, the preferred substituents for group X are a halogen atom (fluorine, chlorine, bromine or iodine), a $C_1$ to $C_6$ alkyl group (methyl, ethyl, propyl, butyl, pentyl or hexyl) or an acyl group ($CH_3COO$-).

The process of the invention can be practised well for compounds in which p > 4, however, compounds in which p = 4 or less are preferred. Of increasing preference are those compounds in which p = 3, p = 2, and p = 1 and compounds wherein p = 0 are most preferred. Non-symmetric compounds (Ar ≠ Ar') can be easily produced by the process of the invention, however it is particularly preferred for symmetric compounds.

The inventors have determined that lignans having at least one hydroxyl group and/or at least one methoxy group on the aromatic residues are particularly effective as pharmaceuticals, thus processes producing compounds in which m ≥ 1 and/or n ≥ 1 are especially preferred. Of increasing preference are those processes producing compounds wherein m = 4, m = 3, m = 0 and m = 2 and those wherein n = 4, n = 3, n = 0 and n = 2. Processes producing compounds in which n = m = 1 or (m + n) = 2 are the most preferred. The substituents RO- and HO- are preferably either in the 4- or the 3-positions on the Ar groups.

In regard to processes producing those compounds containing R, R' and R" groups, methyl, ethyl, propyl, butyl, pentyl, hexyl and phenyl groups are specifically preferred, and of these, methyl groups are the most preferred. These groups may be chosen independently of each other, but it is preferred if R' and R" are the same. Most preferred are processes producing compounds wherein R = R' = R" = methyl.

Particular specific groups which are preferred as Ar, or Ar' are:

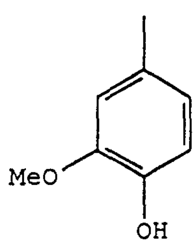
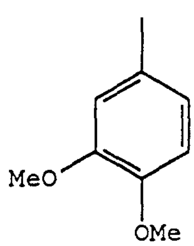
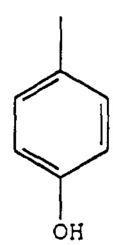
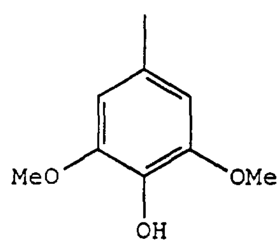

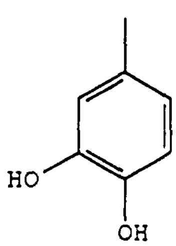
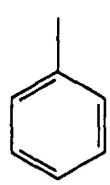
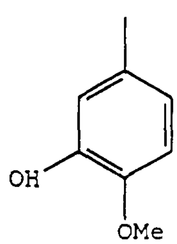
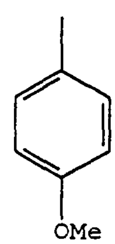

Most preferred is the group:

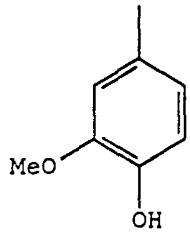

Particular preferred groups for Y and Z are the following:

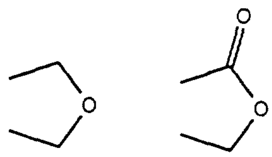

with the former being most preferred. The most preferable processes are those which produce the following compounds:

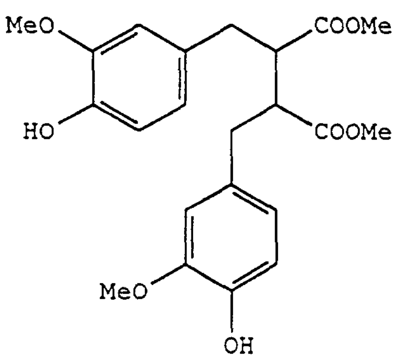

(IIa)

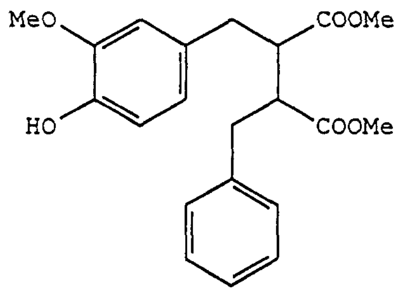

(IIb)

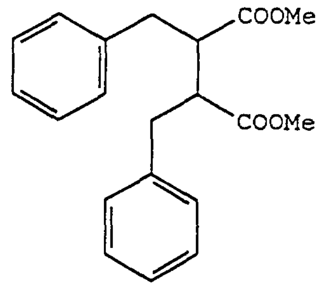

(IIc)

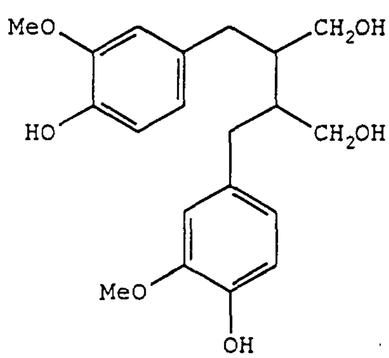

(IIIa)

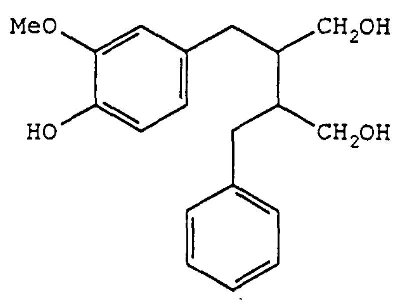

(IIIb)

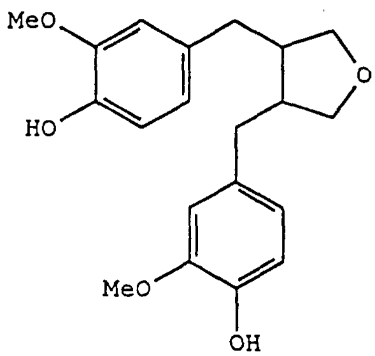

(IVa)

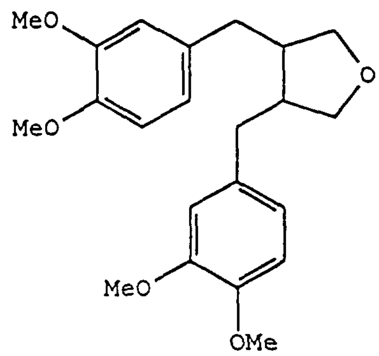

(IVb)

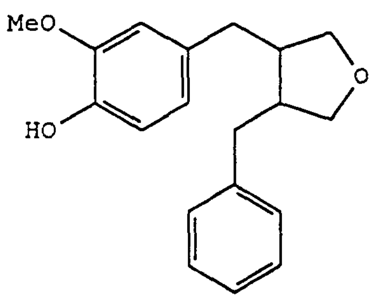

(IVc)

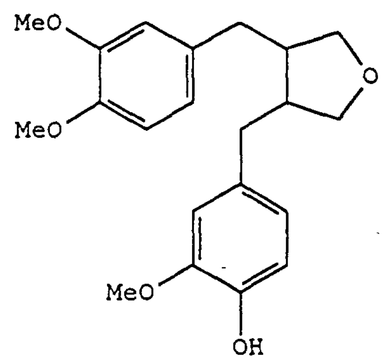

(IVd)

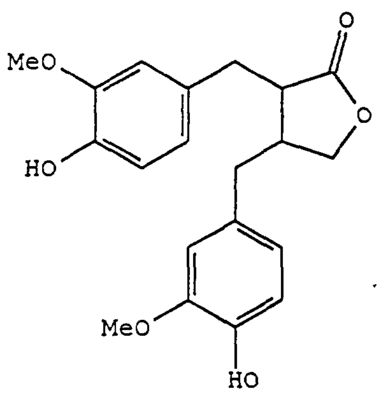

(Va)

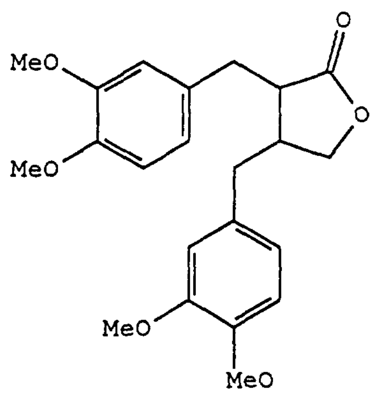

(Vb)

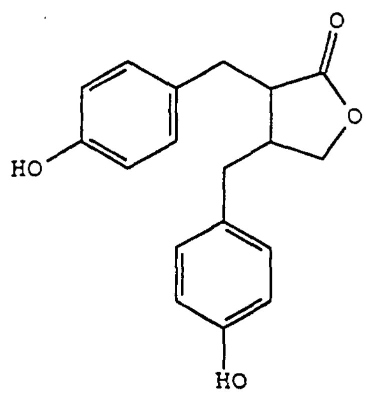

(Vc)

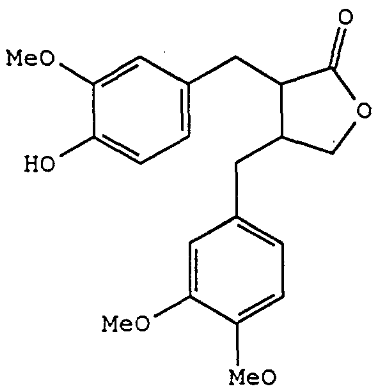
(Vd)

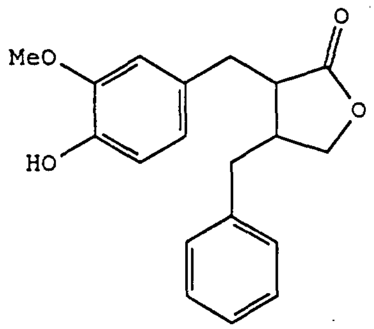
(Ve)

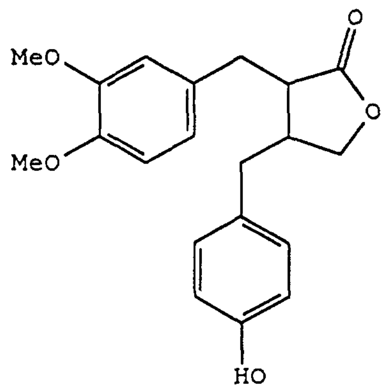
(Vf)

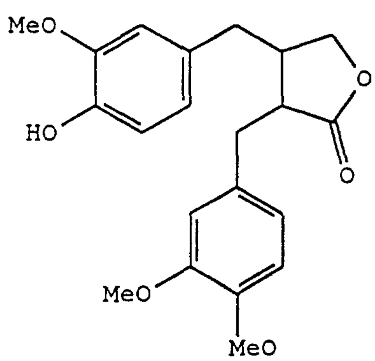
(Vg)

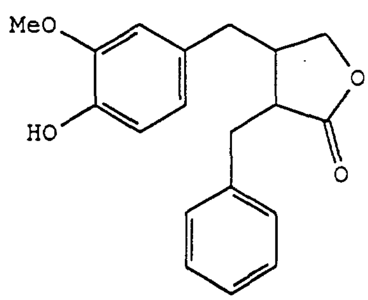
(Vh)

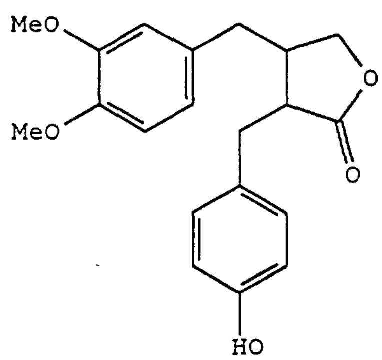
(Vj)

The most preferred process is one in which the compound (IVa) is produced. An overview of the most preferred route is given in Scheme 1. In this case the most preferred process of all, that producing anhydrosecoisolariciresinol is indicated, in which Ar = Ar' = 4-hydroxy-3-methoxyphenyl, along with the most preferred agents for the reactions. In addition, the related methods of production of compounds (II), (III) and (V) are depicted.

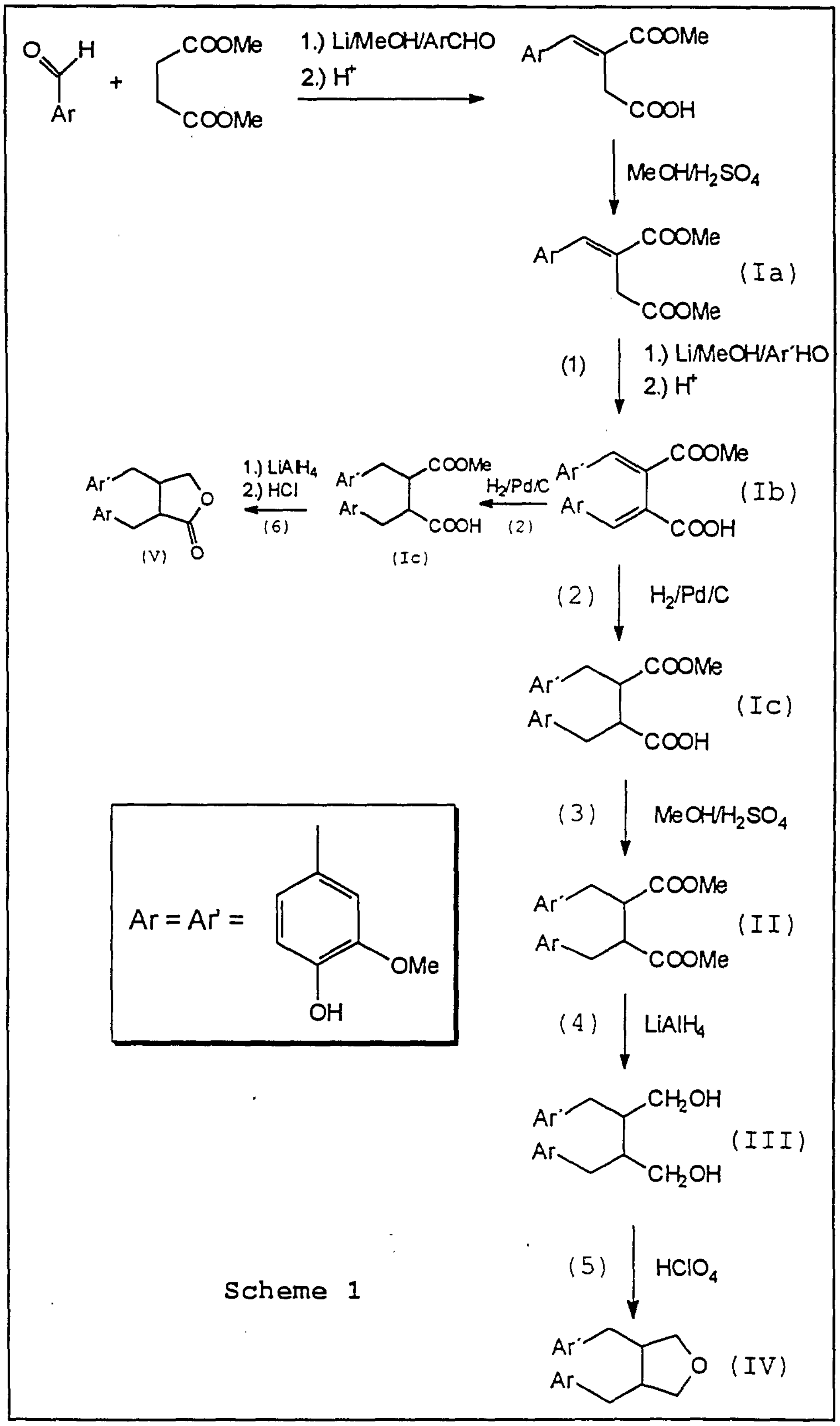


Scheme 1

The most desirable processes in all cases as described above are those in which compounds (Ia) are produced via a Stobbe condensation using Ar-CHO and a diester of succinic acid, in particular using lithium methanolate as a base. However, other reagents and process steps can be utilised if required.

In the process of the present invention both the cis and the trans products are obtained, in each case in a racemic form (i.e. no enantioselectivity is displayed). Where these compounds are depicted throughout the description and claims by chemical structures in which the stereochemistry is not indicated, the structures refer to the individual cis and trans isomers, as well as mixtures thereof and also racemates if a particular isomer of the compound is chiral.

A certain strereoselectivity is observed in the process, in particular during the hydrogenation step, leading in some cases, for example, to a 2:1 excess of one isomer. With the use of Pd on active carbon, in the case of anhydrosecoisolariciresinol production, only the trans product is obtained.

These cis and trans stereoisomers can be separated by any usual method in the art. In the case of cis isomers in which Ar = Ar' the compounds are not chiral and are therefore obtained pure after separation from the trans isomer, and when a particular isomer is chiral, it is obtained as the racemate when separated. The pure optical isomers can be obtained from the racemate by any usual separation method in the art, if desired. In the case of the most preferred compound produced according to the invention, anhydrosecoisolariciresinol, the preferred isomer is the (-)-trans isomer, although the (+)-trans and cis isomers are also desirable.

The products of the present invention are producible by the process of the present invention, and those products include those of formula (I) with the exception of a small number which are already known in the art. Thus the compounds of the present invention are defined by formula (I') as described above.

When $p > 0$, the preferred substituents for group X are a halogen atom (fluorine, chlorine, bromine or iodine), a $C_1$ to $C_6$ alkyl group (methyl, ethyl, propyl, butyl, pentyl or hexyl) or an acyl group ($CH_3COO$-). The invention can be practised well for compounds in which $p > 4$, however, compounds in which $p = 4$ or less are preferred. Of increasing preference are those compounds in which $p = 3$, $p = 2$, and $p = 1$ and compounds wherein $p = 0$ are most preferred. Non-symmetric compounds ($Ar \neq Ar'$) are also included, however symmetric compounds are particularly preferred.

As discussed above, the inventors have determined that lignans having at least one hydroxyl group and/or at least one methoxy group on the aromatic residues are particularly effective as pharmaceuticals, thus compounds in which $m \geq 1$ and/or $n \geq 1$ are especially preferred. Of increasing preference are those compounds wherein $m = 4$, $m = 3$, $m = 0$ and $m = 2$ and those wherein $n = 4$, $n = 3$, $n = 0$ and $n = 2$. Compounds in which $n = m = 1$ or $(n + m) = 2$ are the most preferred. The substituents RO- and HO- are preferably either in the 4- or the 3-positions on the Ar groups.

Those compounds particularly preferred are ones in which the groups Y and Z are COOR', $CH_2OH$ or taken together are groups of the formula:

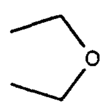 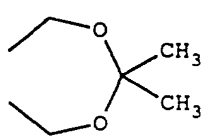

Particular specific groups which are preferred as Ar, or Ar' are:

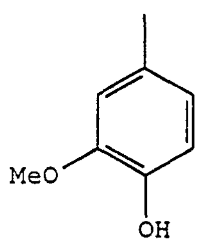 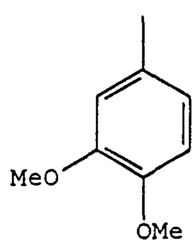 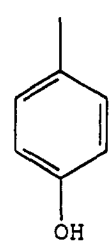 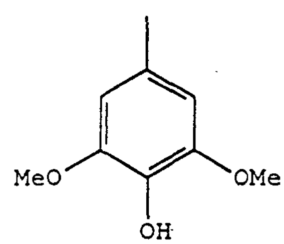

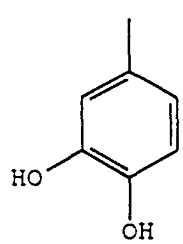 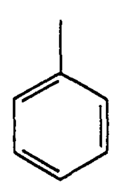 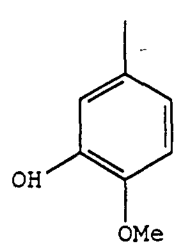 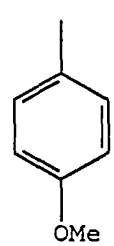

Compounds more particularly preferred are those in which Ar' in formula (I') is a group of formula:

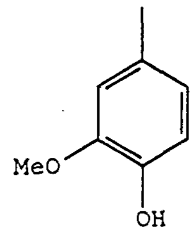

and either:

Ar is a group of the following formula:

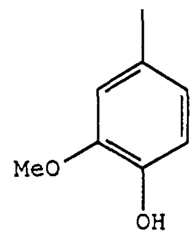

and Y and Z are both COOR' groups, or together form group of formula

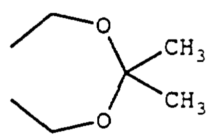

or:

Ar is a group of formula:

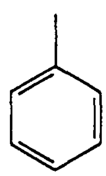

and Y and Z are both COOR' groups, or both a $CH_2OH$ group, or together form a group of formula:

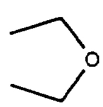

wherein when Y and Z are COOR', the groups R' may be the same or different and are $C_1$ to $C_6$ alkyl groups or phenyl groups. Compounds of this type in which Y = Z = COOR' are especially preferred.

In regard to any preferred compounds as described above containing R, R' and R" groups, methyl, ethyl, propyl, butyl, pentyl, hexyl and phenyl groups are specifically preferred, and of these, methyl groups are the most preferred. These groups may be chosen independently of each other, but it is preferred if R' and R" are the same. Most preferred are compounds wherein R = R' = R" = methyl.

The compounds most preferred of the present invention are those listed below:

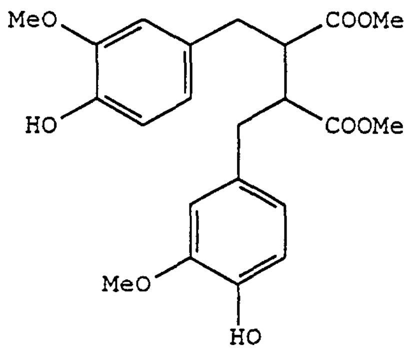

(IIa)

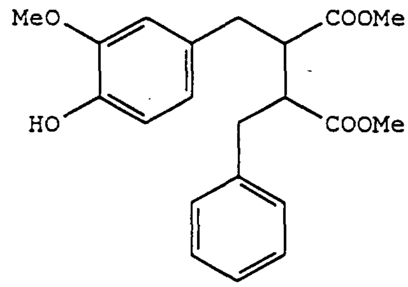

(IIb)

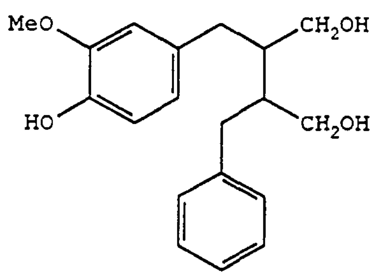

(IIIb)

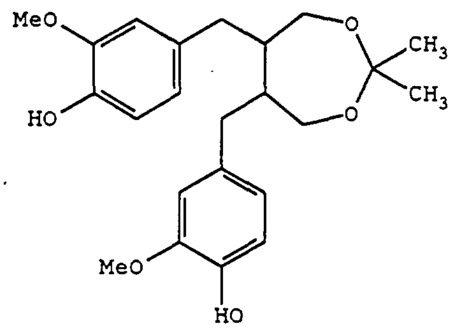

(III'a)

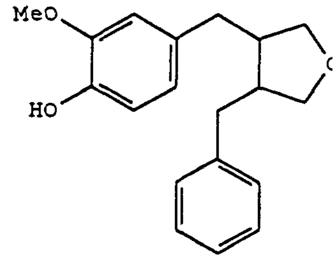

(IVc)

The preferred pharmaceutical compositions of the invention, may contain any one or more of the preferred compounds as described above. Particularly preferred are those which contain one or more of the compounds (IIa), (IIb), (IIIa), (III'a) and (IVc). The compositions may contain any of the pharmaceutically acceptable carriers normal in the art and are thus not particularly limited in this respect.

Any of the compounds (I) producible by the process of the present invention may be used to manufacture medicaments which are effective as SHBG inhibiting agents, anti-tumour agents, agents against prostate cancer and agents against benign prostatic hyperplasia. In the manufacture of a medicament as an anti-tumor agent, the compounds (I) wherein Ar and Ar' both represent independently from each other a phenyl group, a m- or p-methoxy phenyl group or a m,p-dimethoxy phenyl group are excluded. Particularly preferred compounds include all those mentioned above in regard to the preferred processes and products of the invention.

Preferred administration forms of the medicaments include tablet forms, i.v. injectable forms and suppositories.

**Synthesis: Example 1, (±)-anhydrosecoisolariciresinol, (IVa) (see also Scheme 1)**

Production of compound of type (Ia) :
7.6 g (50 mmol) Vanillin (4-hydroxy-3-methoxybenzaldehyde) were dissolved in dried methanol with 7.3 g (50 mmol) succinic acid. 1 g (140 mmol) lithium was added to the solution. The lithium dissolved with evolution of hydrogen and heat and an orange colour developed. After 1.5 hours a further 1.1 g (157 mmol) lithium was added and the mixture was heated under reflux. After two days the resulting yellow precipitate was filtered off, dissolved in water, brought to pH 3 with 32 % hydrochloric acid and extracted with acetic acid. The organic phase was dried with sodium sulphate and placed under a vacuum. Final drying was carried out overnight under high vacuum. 13.3 g of a light yellow powder (a half ester) was obtained (82 % yield).

9.32 g (35 mmol) of the half-ester were dissolved in 200 ml dried methanol. After addition of 0.2 ml 96 % sulphuric acid the mixture was heated to reflux over two days. To the cooling solution one spatula (ca. 1 g) sodium hydrogencarbonate was added and it was allowed to cool. The solvent was removed under vacuum and the solids were separated with water and acetic acid. the organic phase was dried over sodium sulphate. After recrystallisation from diethylether 7.85 g (28 mmol) of a white powder (a diester) were obtained (80 % yield).

Step (1) (production of compound of type (Ib)):

6.19 g (22 mmol) of the diester and 3.35 g (22 mmol) Vanillin were dissolved in 200 ml dried methanol. Then 1.5 g (0.21 mmol) lithium was added. After the reaction tapered off (1.5 h, red colouring) a further 6 g lithium was added in portions over 5 h and the mixture was heated under reflux over 2 days, producing a yellow precipitate. The solvent was removed and the precipitate taken up in 200 ml water. The aqueous red-brown solution was washed with 150 ml methylene chloride and subsequently neutralised with hydrochloric acid. In the aqueous phase a pH of 8 was set up with sodium hydrogencarbonate and extraction was carried out using methylene chloride. After acidification of the aqueous phase with hydrochloric acid (pH 2-3), the half ester was obtained as a yellow precipitate. The crystalline product was dried overnight under high-vacuum and 6.23 g (16 mmol) yellow powder was obtained (77 % yield).

Step (3) (steps (2) and (3) being reversible):

5.12 g (12.8 mmol) of the half-ester were dissolved in 150 ml dried methanol and 0.1 ml sulphuric acid were added. The reaction deposits were heated under reflux for two days. After addition of a spatula of sodium hydrogencarbonate, the solvent was removed and the solids separated with water and diethylether. The organic phase was dried over sodium sulphate and the solvent removed. A yellow-brown raw product was obtained. This could be purified through recrystallisation from toluene giving 4.62 g (11 mmol) of the diester (85 % yield).

Step (2) (production of compound of type (II)):

2.02 g (4.8 mmol) of the diester were stirred in methanol with 200 mg Pd/C (5 % Aldrich) for 1 day under 2 atmospheres pressure of hydrogen. 1.79 g (4.3 mmol) of the saturated diester were obtained (89 % yield).

Step (4) (production of compound of formula (III)):

0.35 g (0.84 mmol) lithium aluminiumhydride were suspended in 50 ml dried THF. The suspension was cooled to -20 °C and 1 g (2.3 mmol)of the saturated diester in 50 ml THF was slowly added over 15 mins. The reaction mixture was brought to room temperature within two hours and stirred for a further two hours. After renewed cooling to 0 °C, 20 ml ethyl acetate were added and the mixture was stirred for 30 mins. and finally hydrolysed with 50 ml 2M hydrochloric acid. The THF was removed in a rotary evaporator, the aqueous phase extracted twice with 50 ml ethyl acetate and the purified organic phase was washed with saturated NaCl solution. 0.26 g (0.72 mmol) of the diol were obtained (86 % yield).

Step (5) (production of compound of type (IV):

100 mg (0.27 mmol) of the diol were dissolved in 20 ml acetone and heated under reflux for 20 mins. with 0.1 ml 60 % perchloric acid. One spatula of sodium hydrogencarbonate was added to the solution after cooling. After dilution with 50 ml diethylether, the solution was shaken twice with 50 ml sodium hydrogencarbonate solution and once with NaCl solution. The organic phase was dried over sodium sulphate and recrystallised from methanol. 0.08 g (0.23 mmol) of the final product, (±)-anhydrosecoisolariciresinol were obtained (87 % yield).

The total yield for the whole process was determined as approximately 29 %.

**Characterisation NMR data: Example 1.1**

For compound (IVa) and a selection of other compounds produced by the process of the present invention, NMR data are presented below.

Compound (IVa):

(500 MHz)

$^1$H: 2.15 m 2H $w_{h/2}$=15Hz; 2.50 dd 2H J=13.7 J'=7.9; 2.56 dd 2H J=13.7 J'=6.6; 3.52 dd 2H J=8.0 J'=5; 3.83 s 6H OMe; 3.93 dd 2H J=8.0 J'=6; 6.50 d 2H J=2; 6.57 dd 2H J=8 J'=2; 6.80 d 2H J=8

$^{13}$C: 59.30; 46.44; 73.26; 111.03; 111.07; 121.30; 132.30; 143.66; 146.30

Compound (±)-trans-(IIb):

(500MHz)

$^1$H: 2.85-3.05 m 6H; 3.60 s 3H; 3.62 s 3H; 3.79 s 3H; 5.5 s 1H 6.5 d; 6.58 dd 1H; 6.80 d 1H; 7.07 d; 7.2 m; 7.25 m;

$^{13}$C: 36.6; 36.9; 37.7; 38.0; 49.1; 49.5; 51.3; 51.66; 53.0; 53.1; 53.2; 57.2; 57.3; 112.7; 115.6; 115.7; 123.0; 123.1; 127.9; 128.0; 129.8; 129.8; 130.2; 130.4; 131.5; 131.7; 139.7; 145.6; 145.7; 147.8; 175.2; 175.3

Compound (±)-cis-(IIb):

(500MHz)

$^1$H: 2.69-2.91 m 4H; 3.2 m 2H; 3.52 s 3H; 3.53 s 3H; 3.83 s 3H; 5.48 s 1H; 6.59 m 2H; 6.78 d 1H J=8.4Hz; 7.11 2H; 7.18 m 1H; 7.24 m 2H

$^{13}$C: As for trans (±)-(IIb)

Compound (±)-trans-(IIIb):
(500MHz)

$^{1}H$: 1.86 m br 1H; 1.91 m br 1H; 2.54-2.9 m 6H; 3.52 m 2H; 3.82 m 5H; 6.60 d 1H J=1.8 Hz; 6.62 dd 1H J=1.8 Hz J'=7.9 Hz; 6.80 d 1H J=7.9 Hz; 7.16 m 3H; 7.26 m 2H
$^{13}C$: 35.85; 36.18; 43.98; 44.13; 55.86; 60.69; 111.37; 114.17; 121.64;126.01; 128.39; 129.05; 132.40; 140.62; 143.82; 146.43

Compound (±)-cis-(IIIb):
(500MHz)

$^{1}H$: 1.98 m 1H; 2.08 m 1H; 2.62 m 3H; 2.72 dd 1H; 3.51 m 2H
$^{13}C$: As for trans (±)-(IIIb)

**Comparative Synthesis: Example 2**

**(see also Scheme 2)**

The known synthesis from the prior art (according to Brown and Daughan, *Heterocycles*, vol. 26, p. 1169, 1987) which most closely approaches the present synthetic route was carried out for compound (IVa) in order to indicate the improved yield associated with the present process.

Step (a):

A Stobbe condensation was carried out using vanillin and dimethyl succinate in a 1:1 equivalent ratio. 2.6 equivalents of lithium methoxide were added as base, without protecting the aryl hydroxyl groups. The ethylenic half-ester was obtained in 90 % yield.

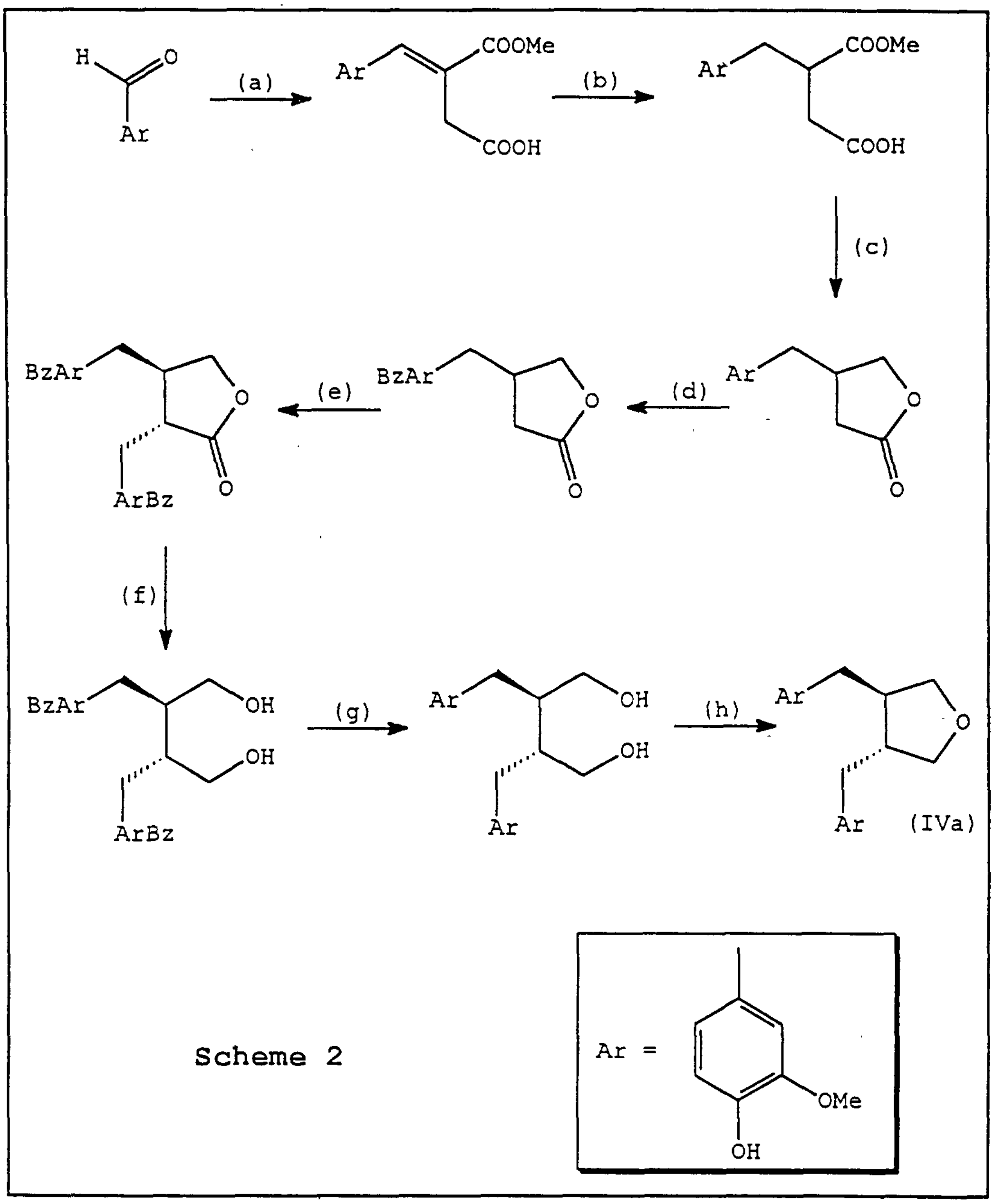


Step (b):

A catalytic hydrogenation of the half-ester was carried out using hydrogen and a palladium/carbon catalyst in the presence of acetic acid. The racemic half-ester was produced in 85 % yield. A separation of the enantiomers was not carried out at this stage, unlike in the prior art process, in order that the yield could be more realistically compared with that of the present process, in which an enantiomer-separation step is not included.

Step (c):

The potassium salt of the racemate was reduced in ethanol using a tenfold excess of $Ca(BH_4)_2$ producing the corresponding butyrolactone derivative in 83 % yield.

Step (d) :

The hydroxyl group of the aromatic ring was transformed into a benzoxy group by reaction with benzyl chloride in chloroform. The product was obtained in 90 % yield.

Step (e):

The lithium anion of the protected lactone was formed using LHDS in THF at -80°C and was reacted with 1 equivalent of 4-benzoxy-3-methoxy-benzylbromide. The product was purified using chromatography and a yield of trans-dialkylated product of 87 % was obtained.

Step (f):

The lactone ring of the trans-dialkylated product was then reduced using $LiAlH_4$ in THF at room temperature to give the corresponding diol in 66 % yield.

Step (g):

A catalytic hydrogenation of the diol using palladium/carbon in ethyl acetate resulted in an 76 % yield of (±)-secoisolarici-resinol.

Step (h):

The resulting diol was dehydrated by means of $HClO_4$ to form the related cyclic ether compound, (±)-anhydrosecoisolariciresinol, (IVa), in 74 % yield, after chromatographic purification.

The total yield for the process was determined as 18 %. Thus this comparative example indicates that the process of the present invention shows a 61 % increase in the available yield in comparison to the closest prior art process, and can be carried out under less stringent conditions in a lesser number of steps.

**Concentration Dependence: Example 3**

In order to determine the strength of the interaction of the compounds produced according to the present invention, tests were carried out investigating remaining SHBG activity to 5α-dihydrotestosterone (DHT) with varying concentrations of the compounds, DHT being a natural hormone that binds with SHBG.

The results for compound (IVa) are depicted in Figure 1. Figure 1 is a graph of the remaining activity of SHBG against the concentration of (IVa). Two different plots appear on the graph, the lower one corresponding to a DHT concentration of 4.6 nM and the upper to a DHT concentration of 9.2 nM. From the latter plot it can be determined that a remaining SHBG activity of 50 % is seen at a concentration of around $1.4*10^{-6}$ M. This compound is therefore the most active, when compared with other preferred compounds (see Table 1). Clearly, as the concentration of (IVa) increases, so the remaining activity of SHBG decreases. As the concentration of DHT is increased, more (IVa) is required to reduce the activity of SHBG by the same amount.

The results of further tests on other preferred compounds are indicated in Table 1. In these experiments a DHT concentration of 9.6 nM was used. From the results it can be seen that compounds (IV) and (V) are particularly effective. Additionally, it is also clear that compounds which possess one MeO and one OH group on the aromatic rings are also very effective.

Table 1

| Compound | Remaining SHBG Activity |
|---|---|
| IIa | 30 % @ $6*10^{-4}$M |
| IIIa | 50 % @ $3.2*10^{-4}$M |
| Va | 50 % @ $8.3*10^{-5}$M |
| IVd | 33 % @ $7*10^{-5}$M |
| IVb | 53 % @ $6.7*10^{-5}$M |
| Vd | 50 % @ $6.9*10^{-5}$M |
| Vb | 82 % @ $6.5*10^{-5}$M |
| Vf | 50 % @ $1.4*10^{-3}$M |
| Vc | 81 % @ $8.4*10^{-4}$M |
| IIC | 61 % @ $7.8*10^{-4}$M |

Table 1 (continued)

| Compound | Remaining SHBG Activity |
|---|---|
| III'a | 75 % @ 6.2*$10^{-4}$M |
| IIb (±)-trans | 35 % @ 1.3*$10^{-4}$M |
| IIb (±)-cis | 95 % @ 1.3*$10^{-4}$M |
| IIIb (±)-trans | 36 % @ 1.6*$10^{-5}$M |
| IIIb (±)-cis | 75 % @ 1.6*$10^{-5}$M |

**Method of inhibition: Example 4**

In order to be able to estimate the association constants of the compounds, it must be determined whether they compete with DHT for the protein, or whether three-species complexes of the type compound-protein-DHT can form. This can be decided using a graph-analogue according to Lineweaver-Burk (Rousseau et *al*, *Nature,* vol. 284, p. 485, 1980).

The specific SHBG activity was thus determined at five differing $^{3}$H-DHT concentrations, each at (IVa) concentrations of 0 M, 2.9*$10^{-5}$ M and 5.8*$10^{-5}$ M. The total binding activity and the unspecific binding activity (USB) were also measured.
The results are outlined in Table 2.

Table 2

| $^{3}$H DHT/nM | Total activity/cpm | USB/cpm | Activity at 0 M IVa | Activity at 2.9*$10^{-5}$ M IVa | Activity at 5.8*$10^{-5}$ M IVa |
|---|---|---|---|---|---|
| 46.0 | 19119 | 938 | 3223 | 1752 | 1446 |
| 18.4 | 16425 | 747 | 2270 | 1231 | 1088 |
| 13.8 | 13080 | 590 | 2075 | 1047 | 836 |
| 9.2 | 8016 | 433 | 1786 | 677 | 518 |
| 4.6 | 4141 | 227 | 928 | 288 | 287 |

The linear regression in a plot of 1/free DHT against 1/bound DHT results in an intersect with the Y-axis and thereby indicates that a competitive "inhibition" of the protein by (IVa) occurs. This graph is depicted in Figure 2.

**Estimation of equilibrium constants $K_a$: Example 5**

With the assumption of competitive binding the estimation of equilibrium constants is possible. Two reactions exist in competition with one another:

$$P + S \overset{K_{a1}}{\rightleftharpoons} PS$$

$$P + I \overset{K_{a2}}{\rightleftharpoons} PI$$

At a concentration of the inhibitor, (**I**), at which the concentration of the protein-substrate complex (**PS**) has reduced by half, the protein-substrate complex concentration becomes equal to the protein-inhibitor concentration, (**PI**). This is the basis for the formula:

$$K_{a2} = \frac{K_{a1}}{\frac{1}{RBA}(1 + R) - R}$$

$K_{a1}$ = association constant DHT/SHBG
$K_{a2}$ = association constant inhibitor/SHBG
R = spec. bound/free DHT at 50% inhibition
RBA = relative binding activity = $DHT_{total}/inhibitor_{total}$ at 50% inhibition

50 % inhibition is reached at a concentration of (IVa) of $1.4^{*}10^{-6}$ M, at a DHT concentration of $9.2^{*}10^{-9}$ M. An association constant of ca. $4^{*}10^{6}$ $M^{-1}$ thereby results.
The association constants for some of the other important compounds are listed in Table 3.

Table 3

| Compound | $K_a$ ($M^{-1}$) |
|---|---|
| IIa | $6.0^{*}10^{4}$ |
| IIIa | $2.0^{*}10^{4}$ |
| Va | $7.7^{*}10^{4}$ |
| IVd | 3.0*105 |
| IVb | $1.0^{*}10^{5}$ |
| Vd | $9.3^{*}10^{4}$ |
| Vb | $0.5^{*}10^{4}$ |
| Vf | $0.5^{*}10^{4}$ |

These results confirm the nature of the data already presented in Table 1.
From the examples in general it can be seen that the compounds producible by the process of the present invention exhibit very favourable binding properties with SHBG, thus having important pharmacological properties.

## Claims

**1.** A process for the production of compounds of formula (I):

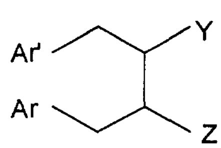

(I)

wherein Ar and Ar' may be the same or different and are represented by groups of the following formula:

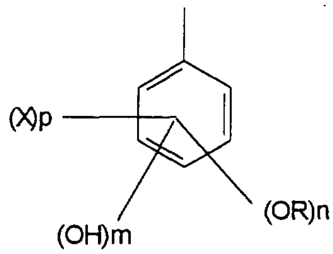

wherein X is a halogen atom, a $C_1$ to $C_6$ alkyl group, an amino group, a nitro group, an acyl group ($CH_3COO$-), a carboxylic acid group or a $C_1$ to $C_6$ alkyl ester of a carboxylic acid, a phenyl group, or a phenyl group substituted with any one of the above groups; R is a $C_1$ to $C_6$ alkyl group; and n, m, and p are integers of 0 to 5, provided that the sum of n, m and p does not exceed 5; and Y and Z may be the same or different and represent COOH, COOR', $CH_2OH$, or $CH_2OR'$ groups or taken together may represent any groups of the following formulae:

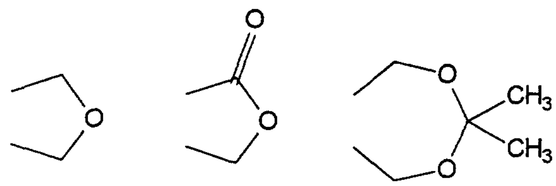

comprising the steps of

(1) Reacting a compound of formula (Ia):

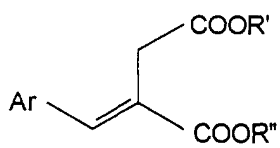

(Ia)

with a lithium/R'OH, or a lithium/R' 'OH mixture in the presence of Ar'-CHO, wherein R' and R" may be the same or different and are each $C_{1-6}$ alkyl or phenyl, to form an intermediate compound (Ib),

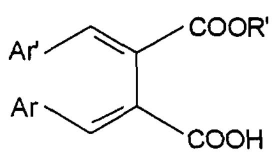

(Ib)

(2) Reducing compounds of formula (Ib), preferably by using hydrogen and a palladium/carbon catalyst, to produce compounds of formula (Ic):

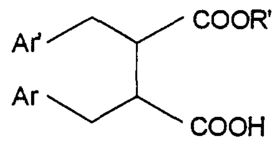

(Ic)

and then optionally performing a further step (3), (6) or (7), or a sequence of steps (3) --> (4) or (3) -->(4) --> (5) given below:

(3) Subjecting compounds (Ic) obtained in step (2) to esterification, preferably by using R' 'OH and sulfuric acid, to produce compounds of formula (II) :

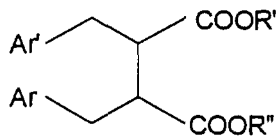

(II)

(4) Reducing the compounds (II) obtained in step (3), preferably by using $LiA1H_4$, to form compounds (III):

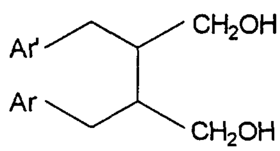

(III)

(5) Subjecting compounds of formula (III) obtained in step (4) to a cyclisation reaction, preferably by using $HC10_4$, to produce compounds of formula (IV):

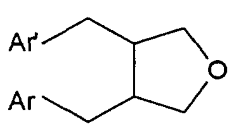

(IV)

(6) Subjecting compounds of formula (Ic) obtained in step (2) to a cyclisation reaction, preferably by using $LiA1H_4$ followed by hydrochloric acid, to form compounds of formula (V):

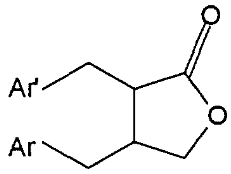

(V)

(7) Subjecting compounds of formula (Ic) obtained in step (2) to an acetal forming reaction in the presence of an acid, to form compounds of formula (III'):

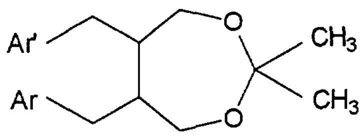

(III')

**2.** The process according to claim 1 wherein R' and R" are each methyl groups.

**3.** The process according to any one of the preceding claims wherein Ar and Ar' are independently selected from the following groups:

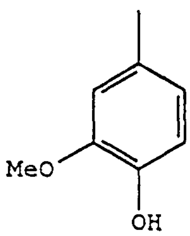
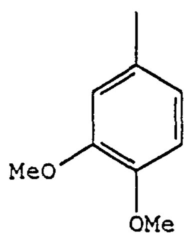
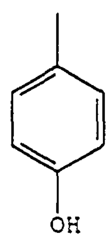
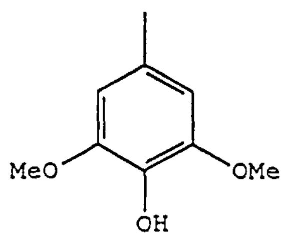

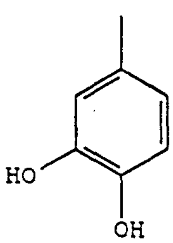
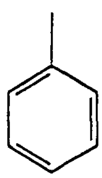
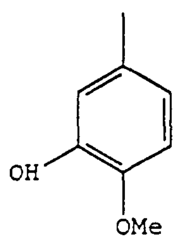
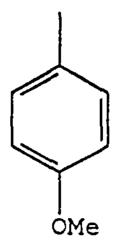

**4.** The process according to claim 1 wherein the following compounds are produced:

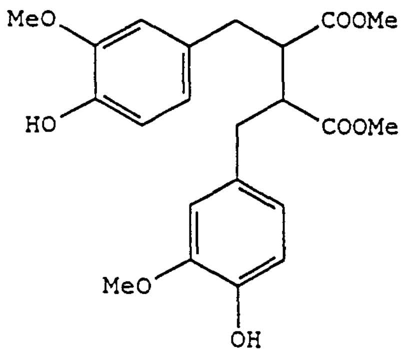

(IIa)

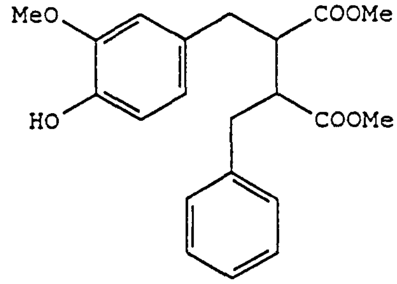

(IIb)

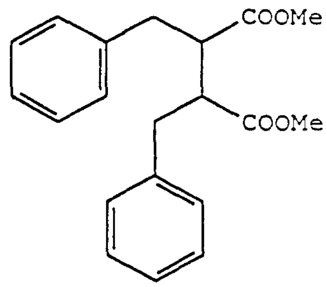

(IIc)

**5.** The process according to claim 1 wherein the following compounds are produced:

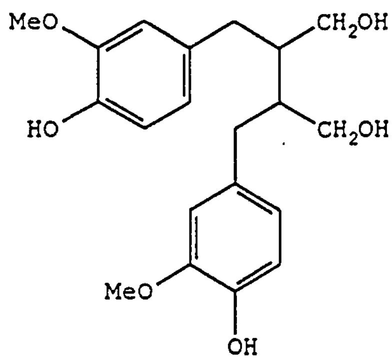

(IIIa)

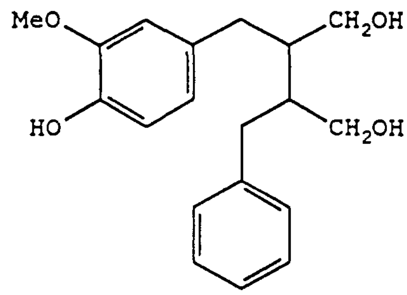

(IIIb)

**6.** The process according to claim 1 wherein the following compounds are produced:

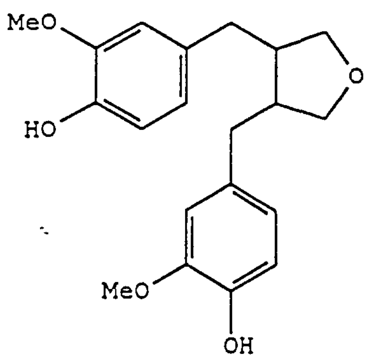

(IVa)

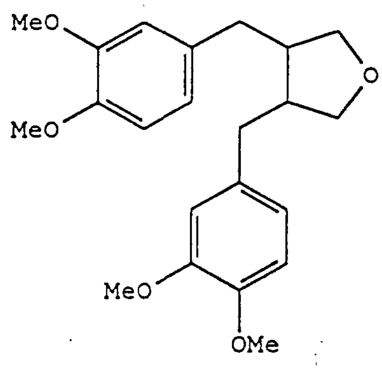

(IVb)

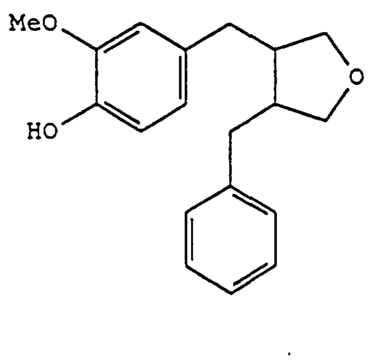

(IVc)

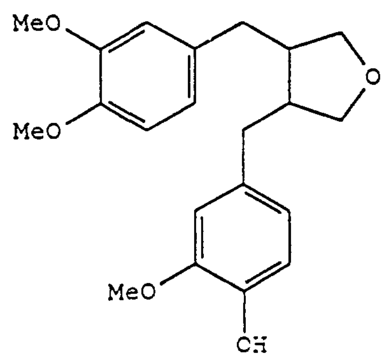

(IVd)

**7.** The process according to claim 1 wherein the following compounds are produced:

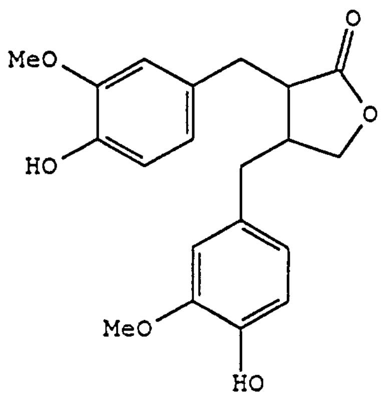

(Va)

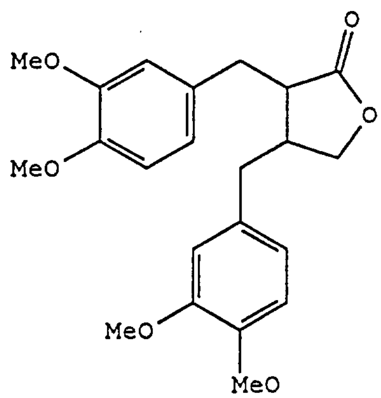

(Vb)

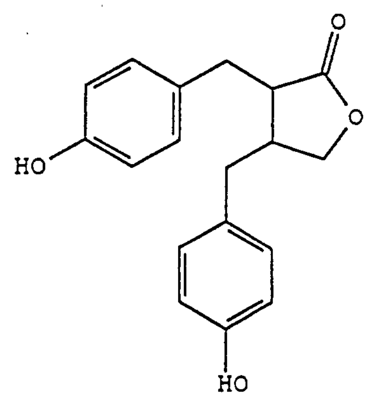

(Vc)

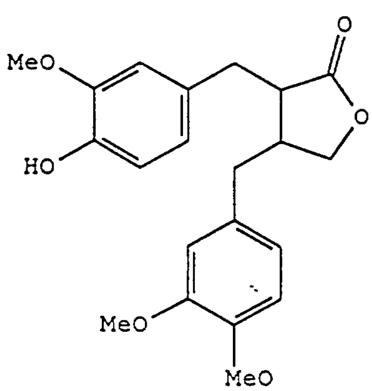

(Vd)

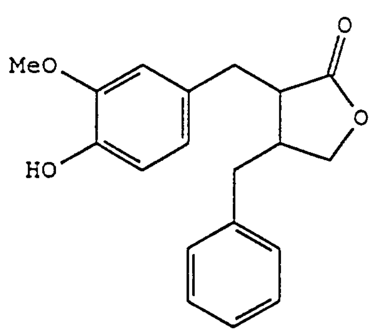

(Ve)

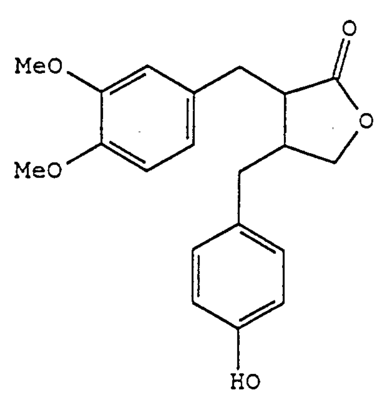

(Vf)

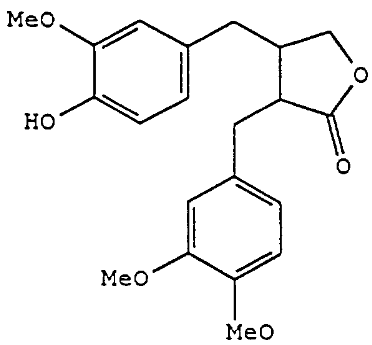

(Vg)

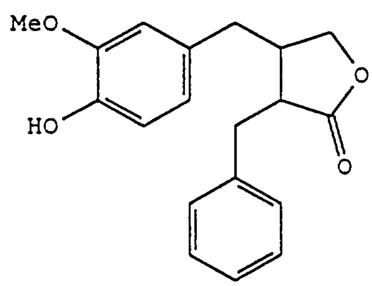

(Vh)

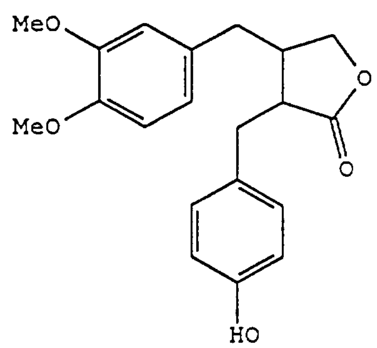

(Vj)

**8.** A compound of the general formula (I'):

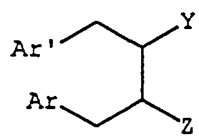

(I')

wherein Ar and Ar' may be the same or different and are represented by groups of the following formula:

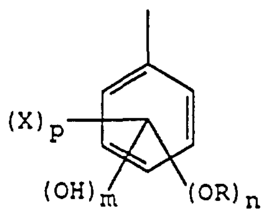

wherein X is a halogen atom, a $C_1$ to $C_6$ alkyl group, an amino group, a nitro group, an acyl group ($CH_3COO$-), a carboxylic acid group or a $C_1$ to $C_6$ alkyl ester of a carboxylic acid, a phenyl group, or a phenyl group substituted with any of the above groups; R is a $C_1$ to $C_6$ alkyl group; and n, m and p are integers of 0 to 5, provided that the sum of n, m and p does not exceed 5; and Y and Z may be the same or different and represent COOH, COOR', $CH_2OH$, or $CH_2OR'$ groups or taken together may represent any groups of the following formulae:

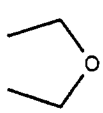

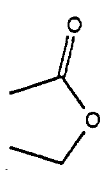

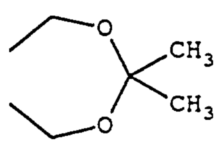

wherein, compounds having p = 0, (m + n) = 3 or less and R = Me are not claimed, except where Ar' is a group of formula:

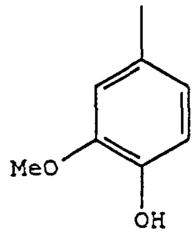

and either:

Ar is a group of the following formula:

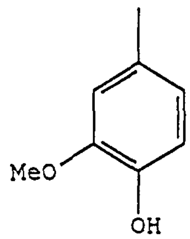

and Y and Z are both COOR' groups, or together form group of formula

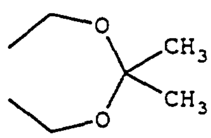

or:

Ar is a group of formula:

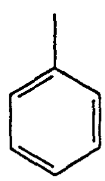

and Y and Z are both COOR' groups, or both a $CH_2OH$ group, or together form a group of formula:

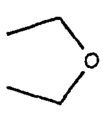

wherein when Y and Z are COOR', the groups R' may be the same or different and are $C_1$ to $C_6$ alkyl groups or phenyl groups.

**9.** Compounds according to claim 8 wherein p = 0, (m + n) = 3 or less and R = Me, wherein if Y and Z are -COOR' groups, Y and Z are identical and R' is a $C_{1-6}$ alkyl group.

**10.** The compounds according to claims 8 or 9 having the following formulae:

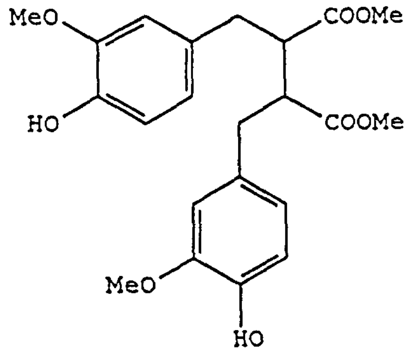

(IIa)

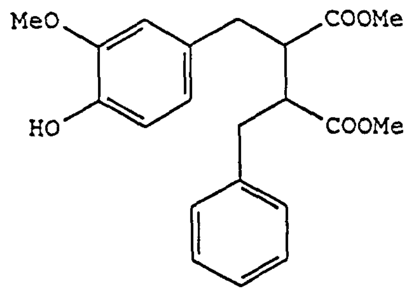

(IIb)

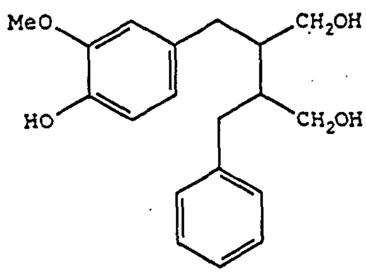

(IIIa)

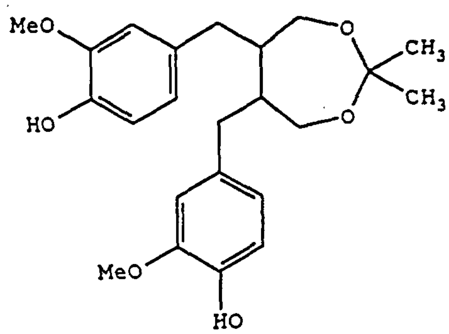

(III'a)

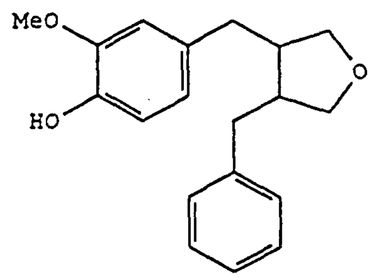

(IVc)

**11.** The use of one or more compounds of formula (I) as defined in claim 1 in the manufacture of a medicament effective as a human sex hormone-binding globuline inhibitor, or as a medicament effective against prostate cancer, or as a medicament effective against benign prostatic hyperplasia.

**12.** The use of one or more compounds of formula (I) as defined in claim 1 in the manufacture of a medicament effective as an anti-tumor agent, with the proviso that those compounds of the formula (I) are excluded, wherein Ar and Ar' both represent indepently from each other a phenyl group, a m - or p-methoxy phenyl group or a m, p-dimethoxy phenyl group.

**13.** The use according to claims 11 or 12, wherein the compounds are those of formulae (II), (III), (IV) or (V) as defined in claim 1.

**14.** The use according to claim 13 wherein the compounds are selected from any one or more of (IIa), (IIb) and (IIc) as defined in claim 4, from any one or more of (IIIa) and (IIIb) as defined in claim 5, from any one or more of (IVa), (IVb) (IVc) and (IVd) as defined in claim 6 or from any one or more of (Va), (Vb), (Vc), (Vd), (Ve), (Vf), (Vg) and (Vj) as defined in claim 7.

**15.** The use according to claim 14 when the compound is anhydrosecoisolariciresinol, (IVa).

**16.** A pharmaceutical composition comprising a pharmaceutically acceptable carrier and one or more compounds selected from those of the general formula (I') as defined in claim 8 and/or from compound (IVa).

**17.** A pharmaceutical composition according to claim 16, wherein the compounds are selected from those as defined in claims 9 or 10.

**Patentansprüche**

1. Verfahren zum Herstellen von Verbindungen der Formel (I):

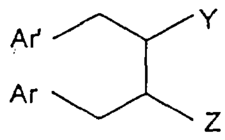

(I)

worin
Ar und Ar' gleich oder verschieden sein können und durch Gruppen der folgenden Formel wiedergegeben werden:

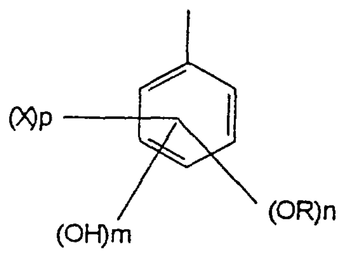

worin

| | |
|---|---|
| X | ein Halogenatom, eine $C_1$ bis $C_6$-Alkylgruppe, eine Aminogruppe, eine Nitrogruppe, eine Acylgruppe ($CH_3COO$-), eine Carbonsäuregruppe oder ein $C_1$ bis $C_6$-Alkylester von einer Carbonsäure, eine Phenylgruppe oder eine mit mindestens einer der vorstehenden Gruppen substituierte Phenylgruppe ist; |
| R | eine $C_1$ bis $C_6$-Alkylgruppe ist, und |
| n, m und p | ganze Zahlen von 0 bis 5 sind, mit der Maßgabe, dass die Summe von n, m und p 5 nicht übersteigt; und |
| Y und Z | gleich oder verschieden sein können und COOH, COOR', $CH_2OH$ oder $CH_2OR'$ -Gruppen bedeuten oder zusammengenommen beliebige Gruppen der folgenden Formeln bedeuten können: |

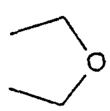

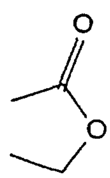

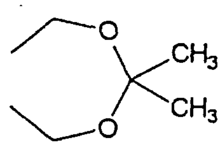

umfassend die Schritte

(1) Umsetzen einer Verbindung der Formel (Ia):

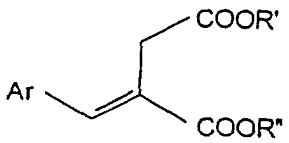

(Ia)

mit einer Lithium/R'OH- oder einer Lithium/R"OH-Mischung in Gegenwart von Ar'-CHO, wobei R' und R" gleich oder verschieden sein können und jeweils $C_{1-6}$-Alkyl oder Phenyl sind, um eine Zwischenverbindung (Ib) zu bilden

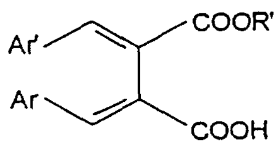

(Ib)

(2) Reduzieren von Verbindungen der Formel (Ib), vorzugsweise unter Verwendung von Wasserstoff und einem Palladium/Kohlenstoff-Katalysator, um Verbindungen der Formel (Ic) herzustellen:

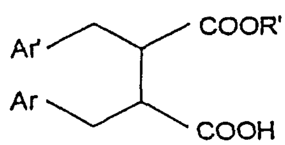

(Ic)

und anschließend gegebenenfalls Durchführen eines weiteren Schritts (3), (6) oder (7), oder einer Sequenz der Schritte (3) → (4) oder (3) → (4) → (5), die nachstehend angegeben sind:

(3) Unterwerfen der in Schritt (2) erhaltenen Verbindungen (Ic) einer Veresterung, vorzugsweise unter Verwendung von R"OH und Schwefelsäure, um Verbindungen der Formel (II) herzustellen:

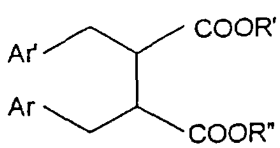

(II)

(4) Reduzieren der in Schritt (3) erhaltenen Verbindungen (II), vorzugsweise unter Verwendung von $LiAlH_4$, um Verbindungen (III) zu bilden:

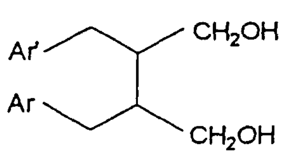

(III)

(5) Unterwerfen der in Schritt (4) erhaltenen Verbindungen der Formel (III) einer Cyclisierungsreaktion, vorzugsweise unter Verwendung von $HClO_4$, um Verbindungen der Formel (IV) herzustellen:

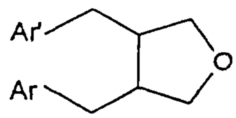

(IV)

(6) Unterwerfen der in Schritt (2) erhaltenen Verbindungen der Formel (Ic) einer Cyclisierungsreaktion, vorzugsweise unter Verwendung von $LiAlH_4$, gefolgt von Chlorwasserstoffsäure, um Verbindungen der Formel (V) zu bilden:

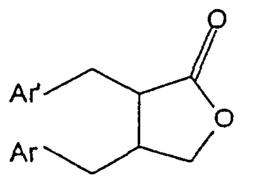

(V)

(7) Unterwerfen von in Schritt (2) erhaltenen Verbindungen der Formel (Ic) einer Acetalbildungsreaktion in Gegenwart einer Säure, um Verbindungen der Formel (III') zu bilden:

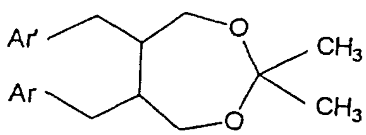

(III')

**2.** Verfahren nach Anspruch 1, wobei R' und R'' jeweils Methylgruppen sind.

**3.** Verfahren nach einem der vorangehenden Ansprüche, wobei Ar und Ar' unabhängig ausgewählt werden aus den folgenden Gruppen:

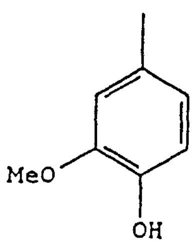
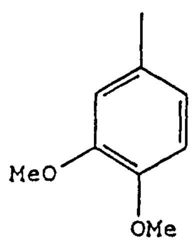
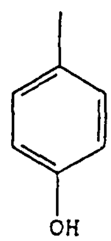
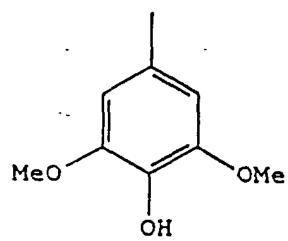

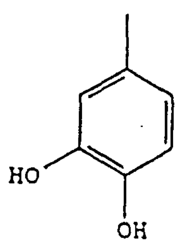
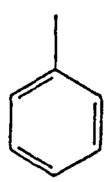
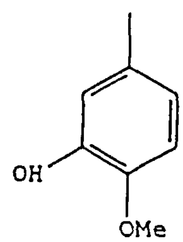
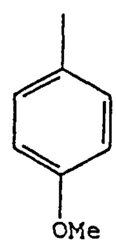

**4.** Verfahren nach Anspruch 1, wobei die folgenden Verbindungen hergestellt werden:

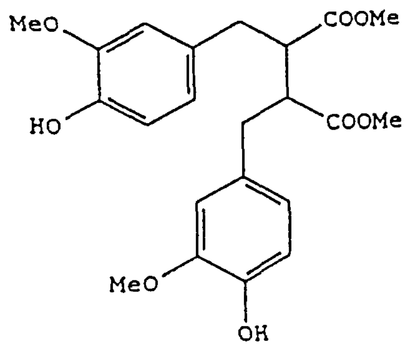

(IIa)

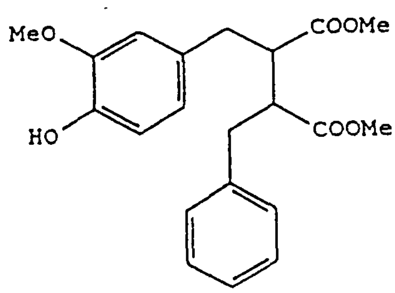

(IIb)

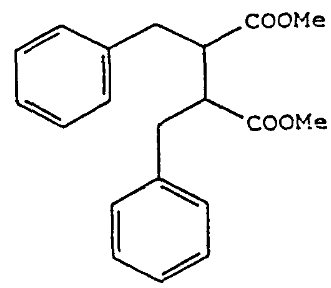

(IIc)

5. Verfahren nach Anspruch 1, wobei die folgenden Verbindungen hergestellt werden:

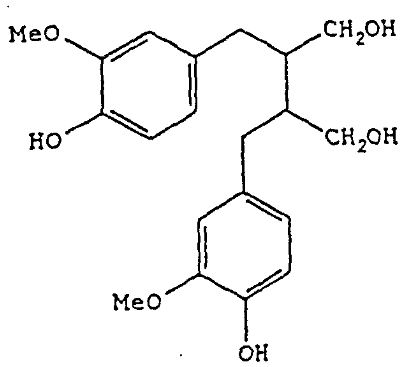

(IIIa)

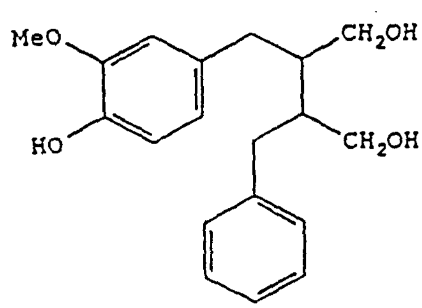

(IIIb)

6. Verfahren nach Anspruch 1, wobei die folgenden Verbindungen hergestellt werden:

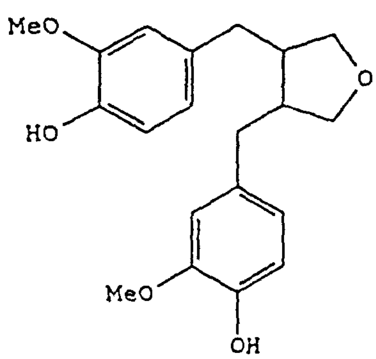

(IVa)

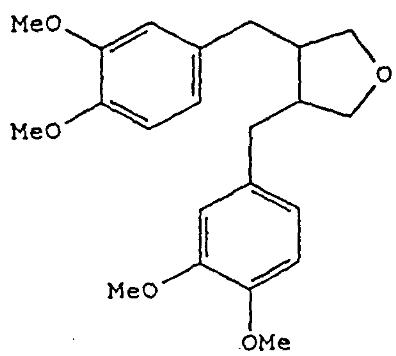

(IVb)

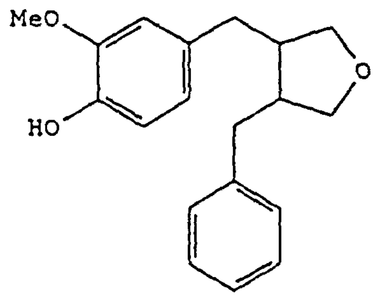
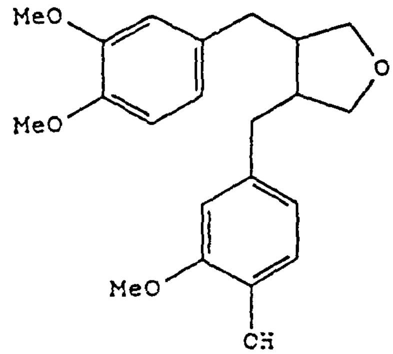

(IVc) (IVd)

7. Verfahren nach Anspruch 1, wobei die folgenden Verbindungen hergestellt werden:

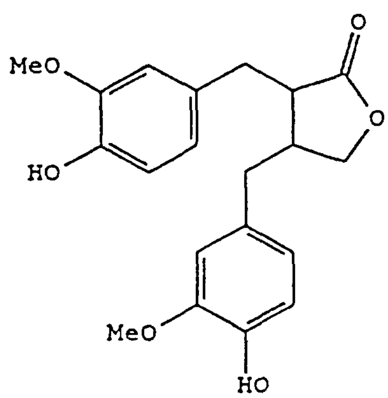
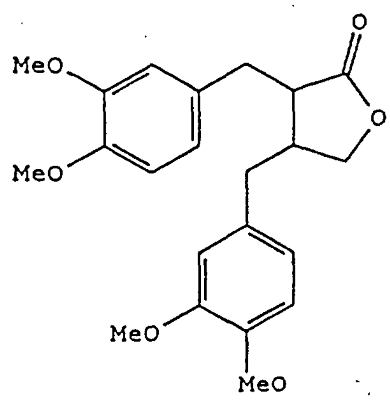
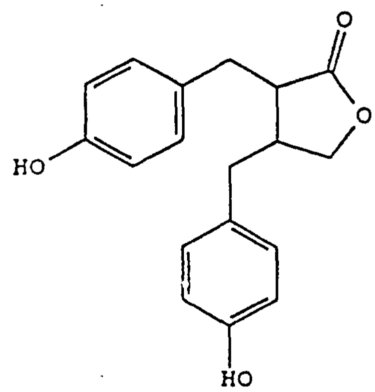

(Va) (Vb) (Vc)

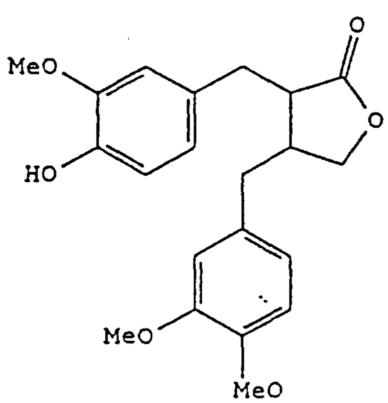
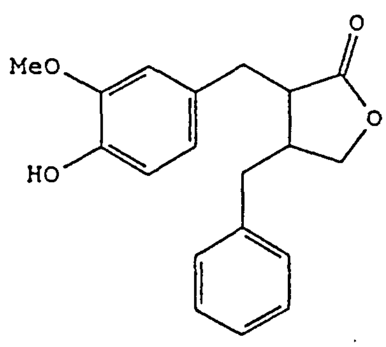
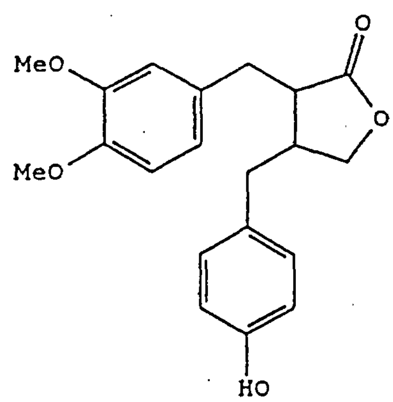

(Vd) (Ve) (Vf)

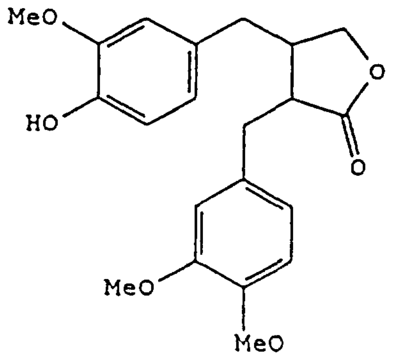

(Vg)

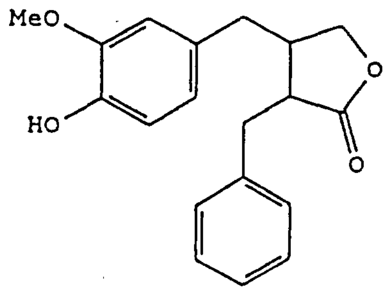

(Vh)

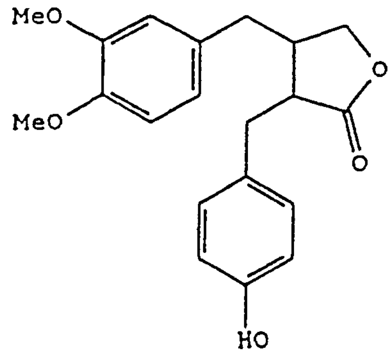

(Vj)

**8.** Verbindung der allgemeinen Formel (I'):

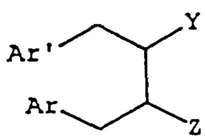

(I')

worin Ar und Ar' gleich oder verschieden sein können und durch Gruppen der folgenden Formel wiedergegeben werden:

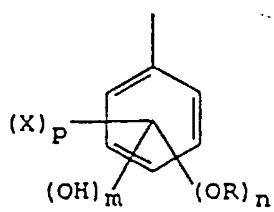

worin

X ein Halogenatom, eine $C_1$ bis $C_6$-Alkylgruppe, eine Aminogruppe, eine Nitrogruppe, eine Acylgruppe ($CH_3COO$-), eine Carbonsäuregruppe oder ein $C_1$ bis $C_6$-Alkylester von einer Carbonsäure, eine Phenylgruppe oder eine mit mindestens einer der vorstehenden Gruppen substituierte Phenylgruppe ist;

R eine $C_1$ bis $C_6$-Alkylgruppe ist; und

n, m und p ganze Zahlen von 0 bis 5 sind, mit der Maßgabe, dass die Summe von n, m und p 5 nicht übersteigt; und

Y und Z gleich oder verschieden sein können und COOH, COOR', $CH_2OH$ oder $CH_2OR'$ -Gruppen bedeuten oder zusammengenommen beliebige Gruppen der folgenden Formeln bedeuten können:

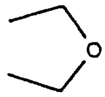 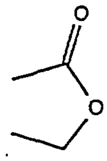 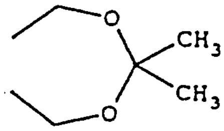

wobei Verbindungen mit p = 0, (m + n) = 3 oder weniger und R = Me nicht beansprucht werden, außer, wenn Ar' eine Gruppe der Formel:

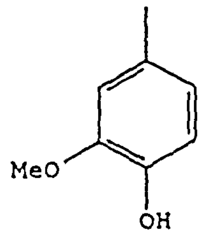

ist und entweder:

Ar eine Gruppe der folgenden Formel ist:

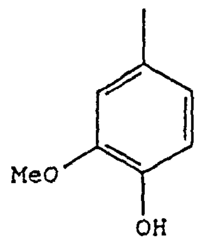

und Y und Z beide COOR'-Gruppen sind oder zusammen eine Gruppe der Formel

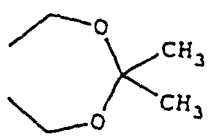

bilden

oder:

Ar eine Gruppe der Formel ist:

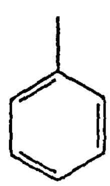

und Y und Z beide COOR'-Gruppen sind oder beide eine $CH_2OH$-Gruppe sind oder zusammen eine Gruppe der Formel:

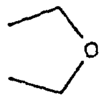

bilden,

wobei, wenn Y und Z COOR' sind, die Gruppen R' gleich oder verschieden sein können und $C_1$ bis $C_6$-Alkylgruppen oder Phenylgruppen sind.

**9.** Verbindungen nach Anspruch 8, wobei p = 0, (m + n) = 3 oder weniger und R = Me, wobei, wenn Y und Z -COOR'-Gruppen sind, Y und Z identisch sind und R' eine $C_{1-6}$-Alkylgruppe ist.

**10.** Verbindungen nach den Ansprüchen 8 oder 9 mit den folgenden Formeln:

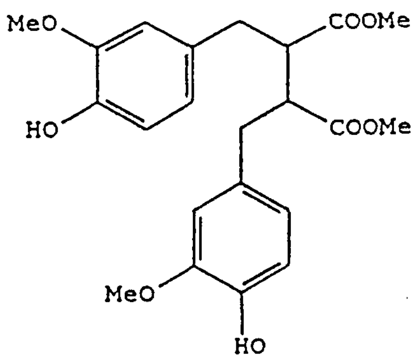

(IIa)

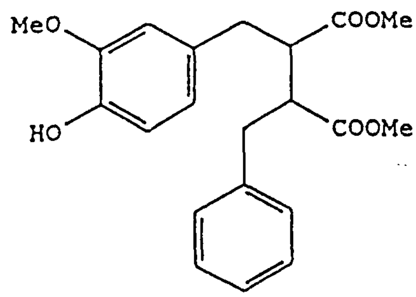

(IIb)

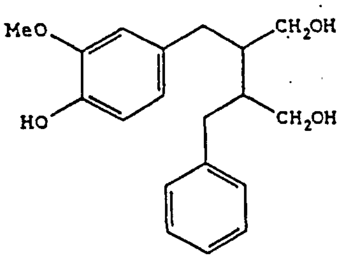

(IIIa)

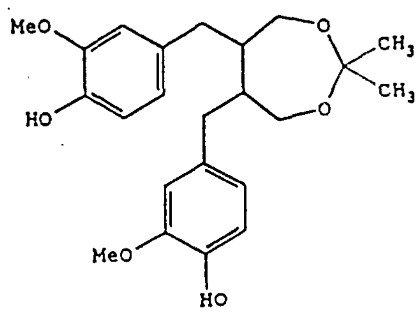

(III'a)

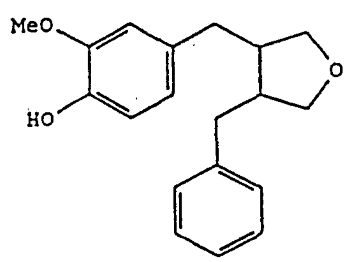

(IVc)

**11.** Verwendung von einer oder mehreren Verbindungen der Formel (I), wie in Anspruch 1 definiert, bei der Herstellung eines Arzneimittels, das als Inhibitor von menschlichem Sexualhormon-bindenden Globulin oder als gegen Prostatakrebs wirksames Arzneimittel oder als gegen gutartige Prostatahyperplasie (Prostatavergrößerung) wirksames Arzneimittel wirksam ist.

**12.** Verwendung von einer oder mehreren Verbindungen der Formel (I), wie in Anspruch 1 definiert, bei der Herstellung eines Arzneimittels, das als Cytostatikum wirksam ist, mit der Maßgabe, dass diejenigen Verbindungen der Formel (I) ausgeschlossen sind, bei denen Ar und Ar' beide unabhängig voneinander eine Phenylgruppe, eine m- oder p-Methoxyphenylgruppe oder eine m, p-Dimethoxyphenylgruppe bedeuten.

**13.** Verwendung nach den Ansprüchen 11 oder 12, wobei die Verbindungen solche der Formeln (II), (III), (IV) oder (V), wie in Anspruch 1 definiert, sind.

**14.** Verwendung nach Anspruch 13, wobei die Verbindungen ausgewählt werden aus einer oder mehreren von (IIa), (IIb) und (IIc), wie in Anspruch 4 definiert, aus einer oder mehreren von (IIIa) und (IIIb), wie in Anspruch 5 definiert, aus einer oder mehreren von (IVa), (IVb), (IVc) und (IVd), wie in Anspruch 6 definiert, oder aus einer oder mehreren von (Va), (Vb), (Vc), (Vd), (Ve), (Vf), (Vg) und (Vj), wie in Anspruch 7 definiert.

**15.** Verwendung nach Anspruch 14, wobei die Verbindung Anhydrosecoisolariciresinol (IVa) ist.

**16.** Pharmazeutische Zusammensetzung, umfassend einen pharmazeutisch annehmbaren Träger und eine oder mehrere Verbindungen, ausgewählt aus den Verbindungen der allgemeinen Formel (I'), wie in Anspruch 8 definiert, und/oder aus Verbindung (IVa).

**17.** Pharmazeutische Zusammensetzung nach Anspruch 16, wobei die Verbindungen aus den Verbindungen ausgewählt werden, die in den Ansprüchen 9 oder 10 definiert sind.

**Revendications**

**1.** Procédé pour la production de composés de formule (I) :

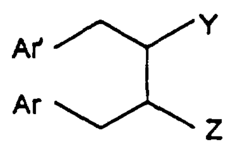

(I)

dans laquelle Ar et Ar' peuvent être identiques ou différents et sont représentés par des groupes de formule suivante :

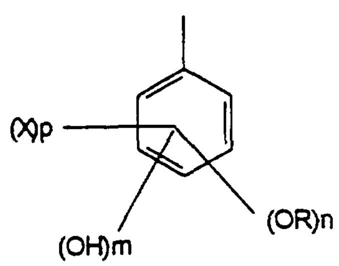

dans laquelle X est un atome d'halogène, un groupe alkyle en $C_1$ à $C_6$, un groupe amino, un groupe nitro, un groupe acyle ($CH_3COO$-), un groupe acide carboxylique ou un ester d'alkyle en $C_1$ à $C_6$ d'un acide carboxylique, un groupe phényle, ou un groupe phényle substitué par l'un quelconque des groupes ci-dessus ; R est un groupe alkyle en $C_1$ à $C_6$ ; et n, m et p sont des entiers de 0 à 5, étant entendu que la somme de n, m et p ne dépasse pas 5 ; et Y et Z peuvent être identiques ou différents et représentent des groupes COOH, COOR', $CH_2OH$ ou $CH_2OR'$ ou, pris ensemble, peuvent représenter tous groupes des formules suivantes :

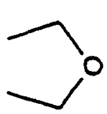 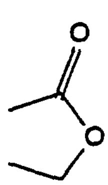 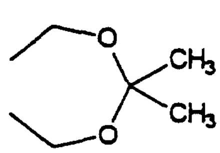

comprenant les étapes consistant

(1) à faire réagir un composé de formule (Ia) :

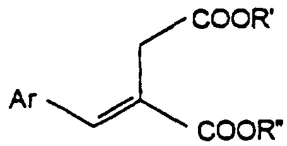

(Ia)

avec un mélange lithium/R'OH ou lithium/R"OH en présence de Ar'-CHO, où R' et R" peuvent être identiques ou différents et sont chacun un groupe alkyle en $C_1$ à $C_6$ ou un groupe phényle, pour former un composé intermédiaire (Ib),

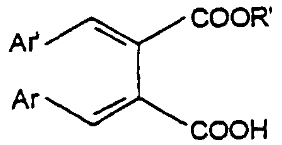

(Ib)

(2) à réduire les composés de formule (Ib), de préférence en utilisant de l'hydrogène et un catalyseur palladium/carbone, pour produire des composés de formule (Ic) :

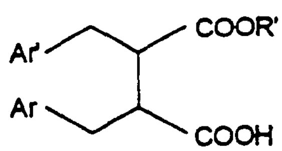

(Ic)

puis à effectuer éventuellement une autre étape (3), (6) ou (7), ou une séquence d'étapes (3) → (4) ou (3) → (4) → (5) présentées ci-après :

(3) à soumettre les composés (Ic) obtenus dans l'étape (2) à une estérification, de préférence en utilisant R' 'OH et de l'acide sulfurique, pour produire des composés de formule (II) :

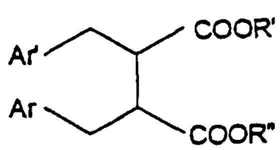

(II)

(4) à réduire les composés (II) obtenus dans l'étape (3), de préférence en utilisant $LiAlH_4$, pour former des composés (III) :

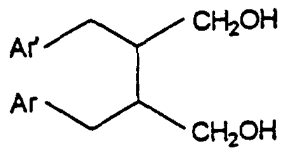

(III)

(5) à soumettre les composés de formule (III) obtenus dans l'étape (4) à une réaction de cyclisation, de préférence en utilisant $HClO_4$, pour produire des composés de formule (IV) :

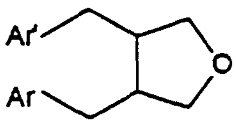

(IV)

(6) à soumettre les composés de formule (Ic) obtenus dans l'étape (2) à une réaction de cyclisation, de préférence en utilisant $LiAlH_4$ puis de l'acide chlorhydrique, pour former des composés de formule (V) :

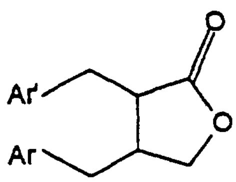

(V)

(7) à soumettre les composés de formule (Ic) obtenus dans l'étape (2) à une réaction de formation d'acétal en présence d'un acide, pour former des composés de formule (III') :

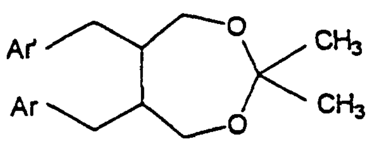

(III')

**2.** Procédé selon la revendication 1, dans lequel R' et R'' sont chacun des groupes méthyle.

**3.** Procédé selon l'une quelconque des revendications précédentes, dans lequel Ar et Ar' sont indépendamment choisis parmi les groupes suivants :

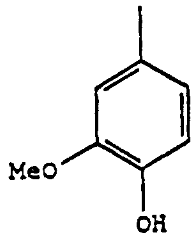
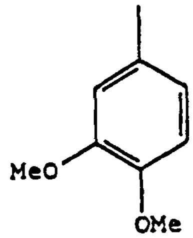
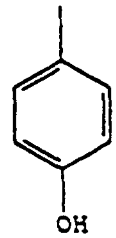
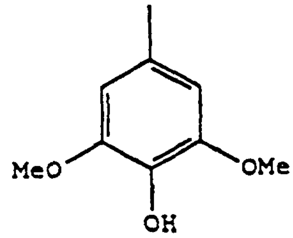

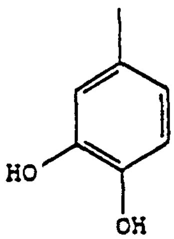
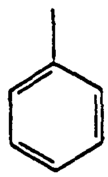
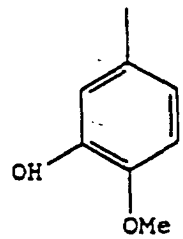
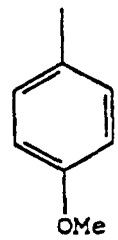

**4.** Procédé selon la revendication 1, dans lequel les composés suivants sont produits :

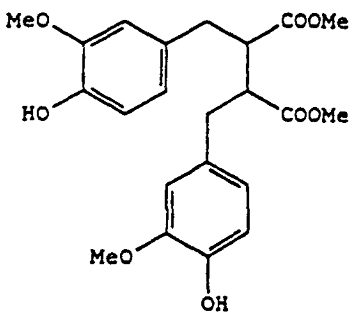
(IIa)

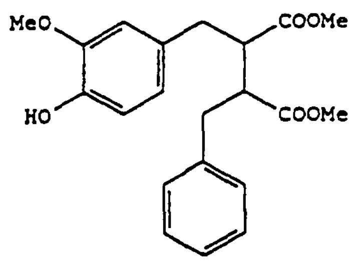
(IIb)

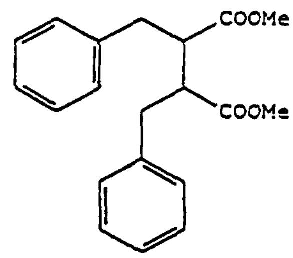
(IIc)

**5.** Procédé selon la revendication 1, dans lequel les composés suivants sont produits :

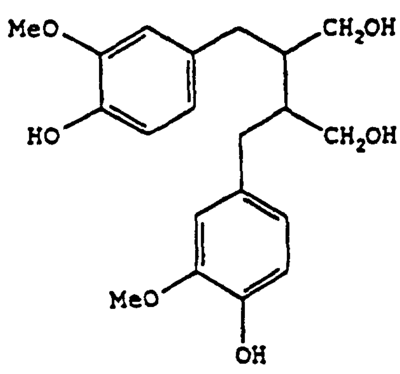
(IIIa)

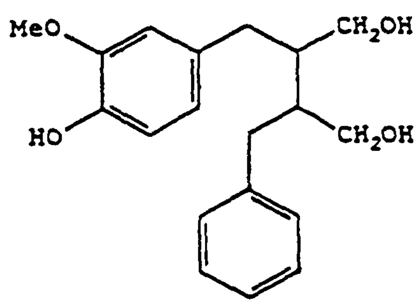
(IIIb)

**6.** Procédé selon la revendication 1, dans lequel les composés suivants sont produits :

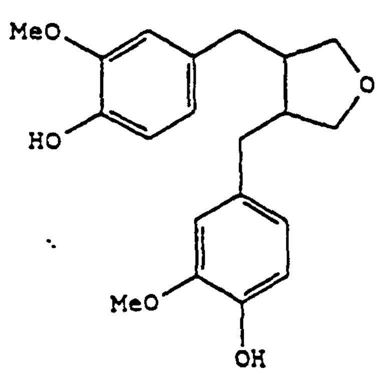

(IVa)

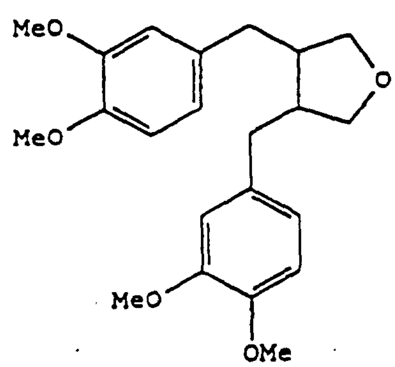

(IVb)

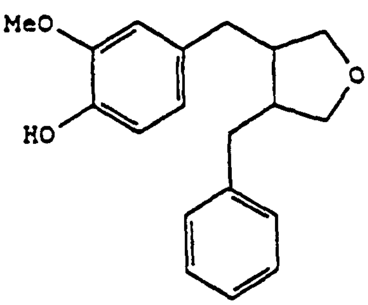

(IVc)

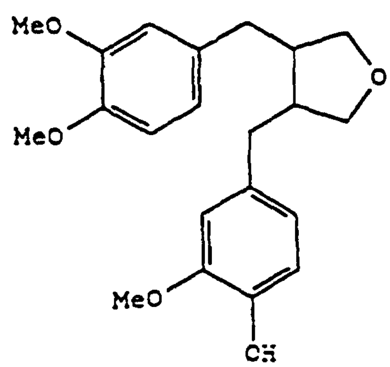

(IVd)

**7.** Procédé selon la revendication 1, dans lequel les composés suivants sont produits :

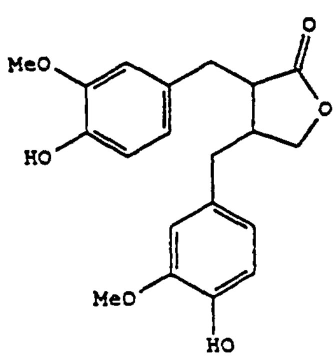

(Va)

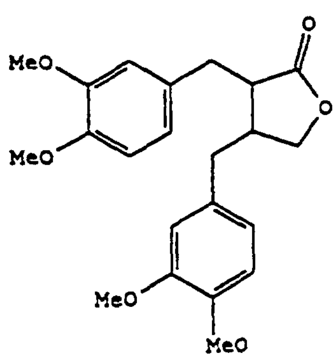

(Vb)

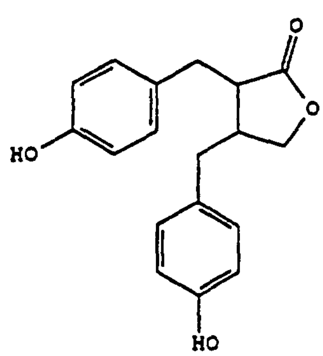

(Vc)

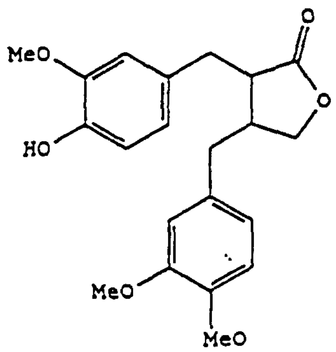 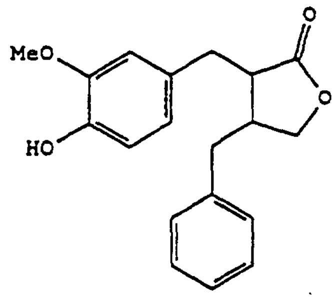 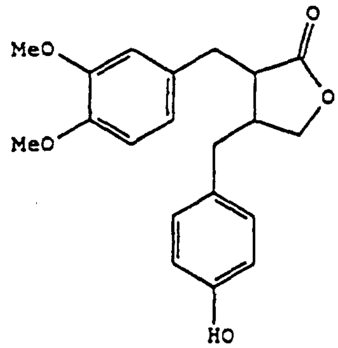

(Vd) (Ve) (Vf)

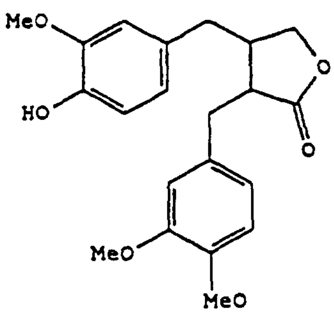 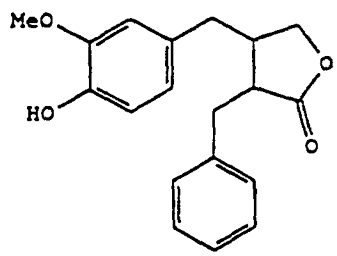 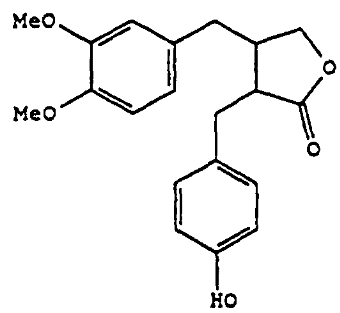

(Vg) (Vh) (Vj)

**8.** Composé de formule générale (I') :

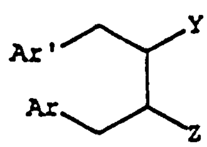

(I')

dans laquelle Ar et Ar' peuvent être identiques ou différents et sont représentés par des groupes de formule suivante :

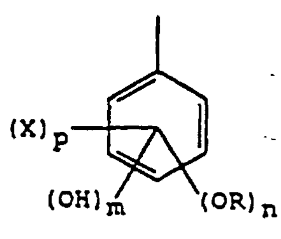

dans laquelle X est un atome d'halogène, un groupe alkyle en $C_1$ à $C_6$, un groupe amino, un groupe nitro, un groupe acyle ($CH_3COO$-), un groupe acide carboxylique ou un ester d'alkyle en $C_1$ à $C_6$ d'un acide carboxylique, un groupe phényle, ou un groupe phényle substitué par l'un quelconque des groupes ci-dessus ; R est un groupe alkyle en $C_1$ à $C_6$ ; et n, m et p sont des entiers de 0 à 5, étant entendu que la somme de n, m et p ne dépasse pas 5 ; et Y et Z peuvent être identiques ou différents et représentent des groupes COOH, COOR', $CH_2OH$ ou $CH_2OR'$ ou, pris ensemble, peuvent représenter tous groupes des formules suivantes :

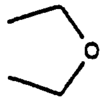 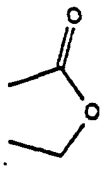 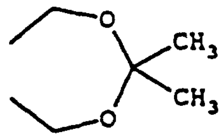

dans laquelle, les composés ayant p=0, (m+n) = 3 ou moins et R=Me ne sont pas revendiqués, sauf lorsque Ar' est un groupe de formule

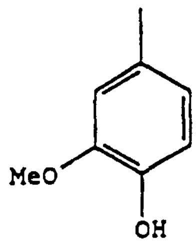

et soit :

Ar est un groupe de formule suivante :

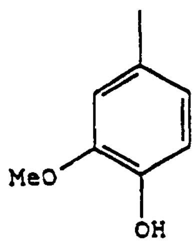

et Y et Z sont tous les deux des groupes COOR', ou forment ensemble un groupe de formule

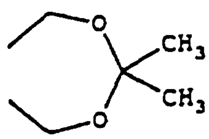

soit :

Ar est un groupe de formule :

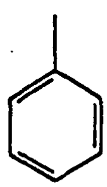

et Y et Z sont tous les deux des groupes COOR', ou tous les deux un groupe $CH_2OH$, ou forment ensemble un groupe de formule :

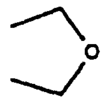

, dans laquelle lorsque Y et Z sont COOR', les groupes R' peuvent être identiques ou différents et sont des groupes alkyle en $C_1$ à $C_6$ ou des groupes phényle.

**9.** Composés selon la revendication 8, dans lesquels p=0, (m+n)=3 ou moins et R=Me, dans lesquels si Y et Z sont des groupes -COOR', Y et Z sont identiques et R' est un groupe alkyle en $C_1$ à $C_6$.

**10.** Composés selon les revendications 8 ou 9, ayant les formules suivantes :

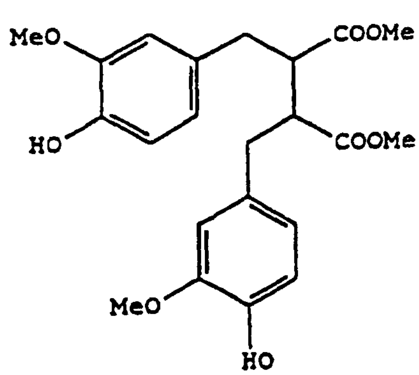

(IIa)

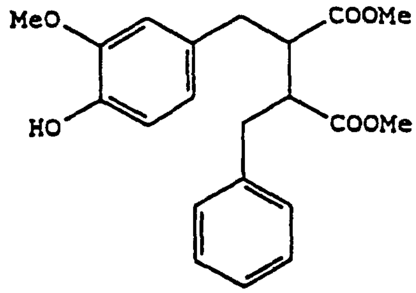

(IIb)

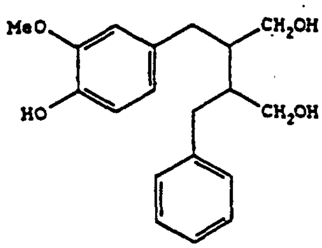

(IIIa)

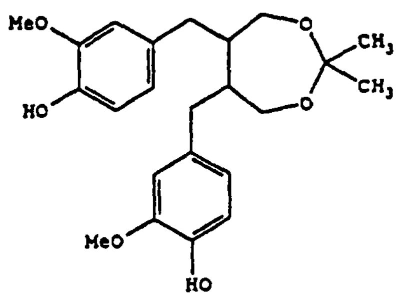

(III'a)

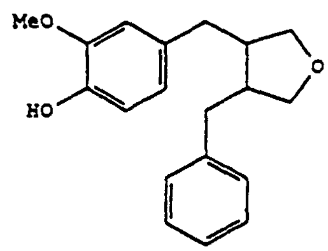

(IVc)

**11.** Utilisation d'un ou plusieurs composés de formule (I) tels que définis dans la revendication 1 dans la fabrication d'un médicament efficace en tant qu'inhibiteur de la protéine porteuse des stéroïdes sexuels humaine, ou en tant que médicament efficace contre le cancer de la prostate, ou en tant que médicament efficace contre l'hypertrophie bénigne de la prostate.

**12.** Utilisation d'un ou plusieurs composés de formule (I) tels que définis dans la revendication 1 dans la fabrication d'un médicament efficace en tant qu'agent antitumoral, à la condition que les composés de formule (I) dans lesquels Ar et Ar' représentent tous les deux, indépendamment l'un de l'autre, un groupe phényle, un groupe m- ou p-méthoxyphényle ou un groupe m, p-diméthoxyphényle, sont exclus.

**13.** Utilisation selon les revendications 11 ou 12, dans laquelle les composés sont ceux des formules (II), (III), (IV) ou (V) tels que définis dans la revendication 1.

**14.** Utilisation selon la revendication 13, dans laquelle les composés sont choisis parmi un ou plusieurs des composés (IIa), (IIb) et (IIc) tels que définis dans la revendication 4, parmi un ou plusieurs des composés (IIIa) et (IIIb) tels que définis dans la revendication 5, parmi un ou plusieurs des composés (IVa), (IVb), (IVc) et (IVd) tels que définis dans la revendication 6 ou parmi un ou plusieurs des composés (Va), (Vb), (Vc), (Vd), (Ve), (Vf), (Vg) et (Vj) tels que définis dans la revendication 7.

**15.** Utilisation selon la revendication 14, dans laquelle le composé est l'anhydrosécoisolaricirésinol (IVa).

**16.** Composition pharmaceutique comprenant un véhicule pharmaceutiquement acceptable et un ou plusieurs composés choisis parmi ceux de formule générale (I') tels que définis dans la revendication 8 et/ou le composé (IVa).

**17.** Composition pharmaceutique selon la revendication 16, dans laquelle les composés sont choisis parmi ceux tels que définis dans les revendications 9 ou 10.

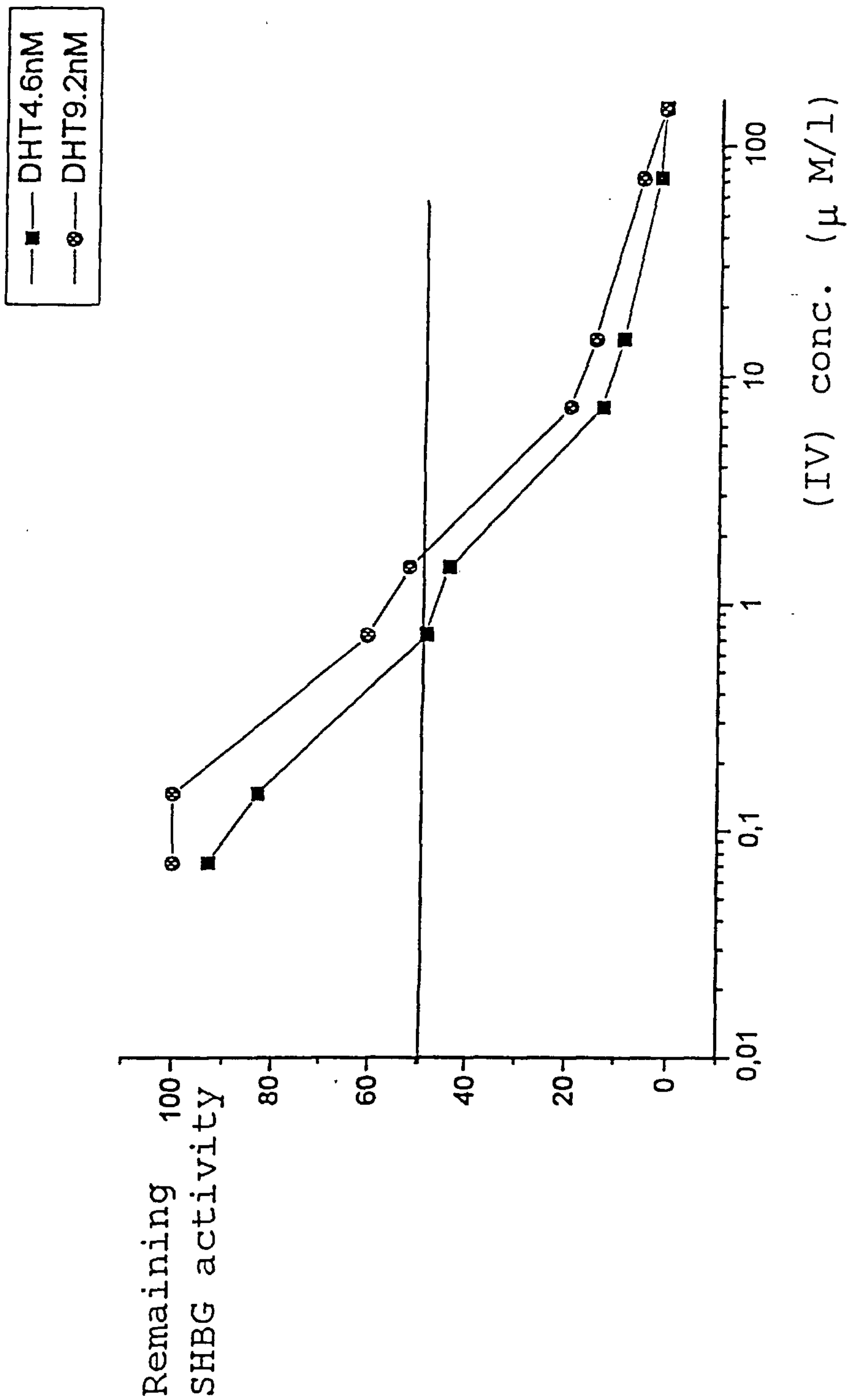

DHT4.6nM
DHT9.2nM
Remaining
SHBG activity
100
80
60
40
20
0
0,01
0,1
1
10
100
(IV) conc. (μ M/l)


Figure 1

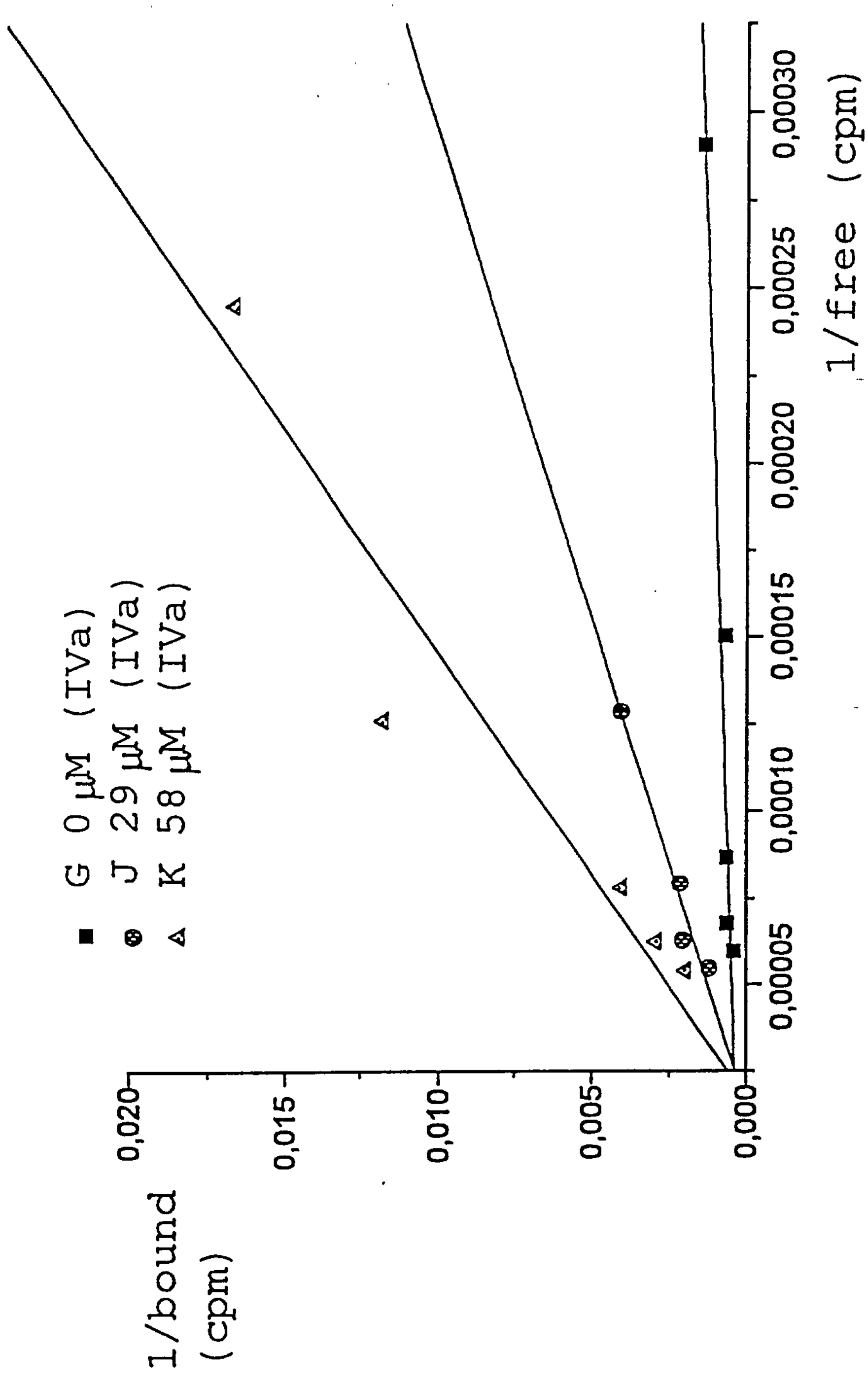
■ G 0 µM (IVa)
⊗ J 29 µM (IVa)
△ K 58 µM (IVa)
1/bound (cpm)
1/free (cpm)
0,000
0,005
0,010
0,015
0,020
0,00005
0,00010
0,00015
0,00020
0,00025
0,00030

Figure 2